# EUROPEAN PATENT SPECIFICATION

(11) **EP 1 521 766 B1**
(45) Date of publication and mention of the grant of the patent: **07.11.2012**
(21) Application number: 03736901.4
(22) Date of filing: 05.06.2003
(51) Int. Cl.: C07H 21/02, C12N 5/10, C12N 15/09, C12P 19/34

(54) **SPLICEOSOME MEDIATED RNA i TRANS /i -SPLICING AND CORRECTION OF FACTOR VIII GENETIC DEFECTS USING SPLICEOSOME MEDIATED RNA TRANS SPLING**
SPLEISSOSOM-VERMITTELTES TRANS-SPLEISSEN VON RNA UND KORREKTUR VON GENETISCHEN FAKTOR-VIII-DEFEKTEN UNTER VERWENDUNG DES SPLEISSOSOM-VERMITTELTEN TRANS-SPLEISSENS VON RNA
TRANS-EPISSAGE D'ARN A MEDIATION PAR SPLICEOSOME (TECHNIQUE SMART) ET CORRECTION DE DEFAUTS GENETIQUES DE FACTEUR VIII A L'AIDE DU TRANS-EPISSAGE D'ARN A MEDIATION PAR SPLICEOSOME

(30) Priority: 05.06.2002 US 386689 P
(43) Date of publication of application: 13.04.2005
(73) Proprietor: VIRxSYS Corporation, Gaithersburg, MD 20877 (US)
(72) Inventor: MITCHELL, Lloyd, G., Bethesda, MD 20814 (US); MANSFIELD, S. Gary, Montgomery Village, MD 20886 (US); PUTTARAJU, Madaiah, Germantown, MD 20876 (US); CLARK, Rebecca, Fort Bragg, NC 28310 (US); GARCIA-BLANCO, Mariano, A., Durham, NC 27713 (US)
(74) Representative: Irvine, Jonquil Claire
(86) International application number: PCT/US2003/017913
(87) International publication number: WO 2003/104412

(56) References cited:
- WO-A-02/053581
- US-A1- 6 013 487
- US-A1- 2003 148 937
- MANSFIELD S G ET AL: "REPAIR OF CFTR MRNA BY SPLICEOSOME-MEDIATED RNA TRANS-SPLICING" GENE THERAPY, MACMILLAN PRESS LTD., BASINGSTOKE, GB, vol. 7, no. 22, November 2000 (2000-11), pages 1885-1895, XP000979769 ISSN: 0969-7128
- VANDENDRIESSCHE THIERRY ET AL: "Long-term expression of human coagulation factor VIII and correction of hemophilia A after in vivo retroviral gene transfer in factor VIII-deficient mice" PROCEEDINGS OF THE NATIONAL ACADEMY OF SCIENCES OF USA, NATIONAL ACADEMY OF SCIENCE. WASHINGTON, US, vol. 96, no. 18, 31 August 1999 (1999-08-31), pages 10379-10384, XP002136830 ISSN: 0027-8424
- CONNELLY S ET AL: "COMPLETE SHORT-TERM CORRECTION OF CANINE HEMOPHILIA A BY IN VIVO GENE THERAPY" BLOOD, W.B. SAUNDERS, PHILADELPHIA, VA, US, vol. 88, no. 10, 15 November 1996 (1996-11-15), pages 3846-3853, XP002926495 ISSN: 0006-4971
- CONNELLY S ET AL: "SUSTAINED PHENOTYPIC CORRECTION OF MURINE HEMOPHILIA A BY IN VIVO GENE THERAPY" BLOOD, W.B. SAUNDERS, PHILADELPHIA, VA, US, vol. 91, no. 9, May 1998 (1998-05), pages 3273-3281, XP002926494 ISSN: 0006-4971
- PUTTARAJU ET AL: 'Spliceosome-mediated RNA trans-splicing as a tool for gene therapy' NATURE BIOTECHNOLOGY vol. 17, no. 3, March 1999, pages 246 - 252, XP002130564
- DE BRASI ET AL: 'Molecular genetics of Hemophilia A' MEDICINA (B AIRES) vol. 56, 1996, pages 509 - 517, XP009053673

## Description

### 1. INTRODUCTION

The present invention relates to methods and compositions for generating novel nucleic acid molecules through targeted spliceosomal mediated trans-splicing. The compositions of the invention include pre-trans-splicing molecules (PTMs) designed to interact with a target precursor messenger RNA molecule (target pre-mRNA) and mediate a trans-splicing reaction resulting in the generation of a novel chimeric RNA molecule (chimeric RNA). The PTMs of the invention have been designed to include features such as (i) binding domains targeted to intron sequences in close proximity to the 3' splice signals of the target intron; (ii) mini-introns; (iii) ISAR (intronic splicing activator and repressor) consensus binding sites; and/or (iv) ribozyme sequences, are more efficiently spliced to the target mRNA.

More particularly, the PTMs of the present invention are genetically engineered to interact with factor VIII (FVIII) target pre-mRNA so as to result in correction of clotting FVIII genetic defects responsible for hemophilia A. The compositions of the invention further include recombinant vector systems capable of expressing the PTMs of the invention and cells expressing said PTMs. The methods of the invention encompass contacting the PTMs of the invention with a FVIII target pre-mRNA under conditions in which a portion of the PTM is trans-spliced to a portion of the target pre-mRNA to form a mRNA molecule wherein the genetic defect in the FVIII gene has been corrected. The methods and compositions of the present invention can be used in gene therapy for correction of FVIII disorders such as hemophilia A.

### 2. BACKGROUND OF THE INVENTION

### 2.1. RNA SPLICING

DNA sequences in the chromosome are transcribed into pre-mRNAs which contain coding regions (exons) and generally also contain intervening noncoding regions (introns). Introns are removed from pre-mRNAs in a precise process called *cis*-splicing (Chow et al., 1977, Cell 12:1-8; and Berget, S.M. et al., 1977, Proc. Natl. Acad. Sci. USA 74:3171-3175). Splicing takes place as a coordinated interaction of several small nuclear ribonucleoprotein particles (snRNP's) and many protein factors that assemble to form an enzymatic complex known as the spliceosome (Moore et al., 1993, in The RNA World, R.F. Gestland and J.F. Atkins eds. (Cold Spring Harbor Laboratory Press, Cold Spring Harbor, N.Y.); Kramer, 1996, Annu. Rev. Biochem., 65:367-404; Staley and Guthrie, 1998, Cell 92:315-326).

In most cases, the splicing reaction occurs within the same pre-mRNA molecule, which is termed *cis*-splicing. Splicing between two independently transcribed pre-mRNAs is termed *trans*-splicing. *Trans*-splicing was first discovered in trypanosomes (Sutton & Boothroyd, 1986, Cell 47:527; Murphy et al., 1986, Cell 47:517) and subsequently in nematodes (Krause & Hirsh, 1987, Cell 49:753); flatworms (Rajkovic et al., 1990, Proc. Nat'l. Acad. Sci. USA, 87:8879; Davis et al., 1995, J. Biol. Chem. 270:21813) and in plant mitochondria (Malek et al., 1997, Proc. Nat'l. Acad. Sci. USA 94:553). In the parasite *Trypanosoma brucei,* all mRNAs acquire a splice leader (SL) RNA at their 5' termini by trans-splicing. A 5' leader sequence is also *trans*-spliced onto some genes in *Caenorhabditis elegans.* This mechanism is appropriate for adding a single common sequence to many different transcripts.

The mechanism of splice leader *trans*-splicing, which is nearly identical to that of conventional *cis*-splicing, proceeds via two phosphoryl transfer reactions. The first causes the formation of a 2'-5' phosphodiester bond producing a 'Y' shaped branched intermediate, equivalent to the lariat intermediate in *cis*-splicing. The second reaction, exon ligation, proceeds as in conventional *cis*-splicing. In addition, sequences at the 3' splice site and some of the snRNPs which catalyze the *trans*-splicing reaction, closely resemble their counterparts involved in *cis*-splicing.

*Trans*-splicing may also refer to a different process, where an intron of one pre-mRNA interacts with an intron of a second pre-mRNA, enhancing the recombination of splice sites between two conventional pre-mRNAs. This type of *trans*-splicing was postulated to account for transcripts encoding a human immunoglobulin variable region sequence linked to the endogenous constant region in a transgenic mouse (Shimizu et al., 1989, Proc. Nat'l. Acad. Sci. USA 86:8020). In addition, *trans*-splicing of c-myb pre-RNA has been demonstrated (Vellard, M. et al. Proc. Nat'l. Acad. Sci., 1992 89:2511-2515) and more recently, RNA transcripts from cloned SV40 *trans*-spliced to each other were detected in cultured cells and nuclear extracts (Eul et al., 1995, EMBO. J. 14:3226). However, naturally occurring *trans-*splicing of mammalian pre-mRNAs is thought to be a rare event (Flouriot G. et al., 2002 J. Biol. Chem*:* Finta, C. et al., 2002 J. Biol Chem 277:5882-5890).

*In vitro trans*-splicing has been used as a model system to examine the mechanism of splicing by several groups (Konarska & Sharp, 1985, Cell 46:165-171 Solnick, 1985, Cell 42:157; Chiara & Reed, 1995, Nature 375:510; Pasman and Garcia-Blanco, 1996, Nucleic Acids Res. 24:1638). Reasonably efficient *trans-*splicing (30% of *cis*-spliced analog) was achieved between RNAs capable of base pairing to each other, splicing of RNAs not tethered by base pairing was further diminished by a factor of 10. Other *in vitro trans*-splicing reactions not requiring obvious RNA-RNA interactions among the substrates were observed by Chiara & Reed (1995, Nature 375:510), Bruzik J.P. & Maniatis, T. (1992, Nature 360:692) and Bruzik J.P. and Maniatis, T., (1995, Proc. Nat'l. Acad. Sci. USA 92:7056-7059). These reactions occur at relatively low frequencies and require specialized elements, such as a downstream 5' splice site or exonic splicing enhancers.

In addition to splicing mechanisms involving the binding of multiple proteins to the precursor mRNA which then act to correctly cut and join RNA, a third mechanism involves cutting and joining of the RNA by the intron itself, by what are termed catalytic RNA molecules or ribozymes. The cleavage activity of ribozymes has been targeted to specific RNAs by engineering a discrete "hybridization" region into the ribozyme. Upon hybridization to the target RNA, the catalytic region of the ribozyme cleaves the target. It has been suggested that such ribozyme activity would be useful for the inactivation or cleavage of target RNA *in vivo*, such as for the treatment of human diseases characterized by production of foreign or aberrant RNA. In such instances small RNA molecules are designed to hybridize to the target RNA and by binding to the target RNA prevent translation of the target RNA or cause destruction of the RNA through activation of nucleases. The use of antisense RNA has also been proposed as an alternative mechanism for targeting and destruction of specific RNAs.

Using the *Tetrahymena* group I ribozyme, targeted trans-splicing was demonstrated in E. coli. (Sullenger B. A. and Cech. T. R. , 1994, Nature 341: 619-622), in mouse fibroblasts (Jones, J. T. et al., 1996, Nature Medicine 2: 643-648), human fibroblasts (Phylacton, L. A. et al. Nature Genetics 18: 378-381) and human erythroid precursors (Lan et al., 1998, Science 280: 1593-1596). For a review of clinically relevant technologies to modify RNA see Sullenger and Gilboa, 2002 Nature 418: 252-8. The present invention relates to the use of targeted trans-splicing mediated by native mammalian splicing machinery, i. e. spliceosomes, to reprogram or alter the coding sequence of a targeted m-RNA.

U. S. Patent Nos. 6,083,702, 6,013,487 and 6,280,978 describe the use of PTMs to mediate a trans-splicing reaction by contacting a target precursor mRNA to generate novel chimeric RNAs. Mansfield et al. in Gene Therapy (2000) 22 (7) 1885-1895 also describe use of such spliceosome-mediated RNA trans-splicing in the treatment of genetic defects such as in the CFTR gene.

The present invention provides specific PTM molecules designed to correct FVIII defective genes. The specific PTMs of the invention may be used to treat a variety of different FVIII disorders such as hemophilia A.

Published International Application WO 02/053581 (in common ownership) is Article 54(3) prior art based on its filing date and also describes trans-splicing to correct defective human FVIII genes, but only teaches replacement of exons 23-26 of human Factor VIII pre-mRNA.

### 2.2. FACTOR VIII GENETIC DEFECTS

Hemophilia A is a genetic defect caused by a deficiency in clotting Factor VIII (FVIII). The deficiency is a sex-linked recessive disorder manifested by frequent spontaneous intra-articular joint and soft tissue bleeding episodes (Roberts and Hoffman, 1995). The phenotype of the FVIII deficiency, which constitutes 80% of all hemophilic patients, is directly related to the levels of functional FVIII circulating in the plasma. For example, patients with less than 1% normal FVIII activity are phenotypically severe with frequent bleeding episodes requiring treatment with either plasma-derived or recombinant FVIII products. Patients with mild disease symptoms maintain ≥ 5%-30% of normal FVIII levels and typically have few spontaneous bleeding episodes, however, such patients are still at risk for trauma-induced bleeding. Factor levels of 1-5% produce intermediate rates of spontaneous bleeding.

Treatment with plasma-purified or recombinant FVIII protein at the time of bleeding is the standard of care for hemophilic patients. Studies demonstrate that prophylactic FVIII infusion regiments, three times a week have a dramatic effect on reducing the rate and severity of joint bleeding (Lofqvist et al., 1997). Thus the goal for FVIII gene transfer is sustained long-term factor production at levels of ≥ 5% that would effectively convert severely affected patients to a milder phenotype.

The biology and biochemistry of FVIII has been extensively reviewed by Kaufman et al., 1997. The FVIII gene encodes a mRNA of 9Kb which is composed of 26 exons. Although cells of the reticuloendothelial system (RES) secrete functional FVIII, the liver is the principal source of synthesis (Wion et al., 1985). In the liver, both sinusoidal endothelial cells and hepatocytes are capable of synthesizing and secreting FVIII (Do et al., 1999; Hollestelle et al., 2001). The FVIII cDNA encodes a single chain polypeptide of 2351 amino acids (Burke et al., 1986), which is proteolytically cleaved to produce a mature 280 KD heterodimer protein comprised of heavy and light chains.

Genetic analysis of hemophilic patients reveals a broad range of different mutations in the FVIII gene. Since the cloning of the FVIII gene, over 2500 hemophilic patients have been examined to determine the genetic basis of their disease. Such studies have identified a correlation between specific types of mutations and the phenotype of the disease. For example, 40% of all severe hemophilic patients carry inversion secondary to intrachromosonal crossing over between exon 1 and 22 (Wacey et al., 1996). The remaining 60% of hemophilic patients have large deletions, frameshift, missense and nonsense mutations. Over 80% of patients with mild-to-moderate disease carry missense mutations. Hence, the human FVIII gene was previously recognized as a target for genetic therapeutic intervention (see e.g. Vandendriessche Thierry et al. PNAS (1999) 96, 10379 - 10384, Connelly et al. Blood (1996) 88, 3846-3853 and Connelly et al. Blood (1998) 91, 3273-3281).

However, the large size of the complete FVIII cDNA (7-9Kb) has limited the ability to design vector systems for delivery of FVIII to tissues *in vivo.* By utilizing spliceosomal mediated RNA trans-splicing technology, FVIII gene correction can be carried out without the need for expressions of the entire FVIII gene thereby circumventing any problems associated with limited vector size.

### 3. SUMMARY OF THE INVENTION

The present invention relates to novel pre-trans-splicing nucleic acid molecules (hereinafter referred to as "PTMs") designed to interact with a natural target human Factor VIII pre-mRNA molecule (hereinafter referred to as "FVIII pre-mRNA") and mediate a spliceosomal trans-splicing reaction resulting in the generation of a novel chimeric RNA molecule (hereinafter referred to as "chimeric RNA"). The novel PTMs of the invention comprise:
a) one or more target-binding domains that target binding of the nucleic acid to a human Factor VIII target pre-mRNA expressed within a cell;
b) (1) a 3' splice region comprising a 3' splice acceptor site, a branch point and a polypyrimidine tract and/or (2) a 5' splice donor site;
c) a spacer region that separates the 3' splice region or 5' splice donor site from the target-binding domain; and
d) a nucleotide sequence of (1) human Factor VIII exons 15 to 26, 16 to 26, 17 to 26, 18 to 26, 19 to 26, 20 to 26, 21 to 26 or 22 to 26, to be trans-spliced to the target pre-mRNA (for mediating 3' exon replacement in the pre-mRNA), or (2) human Factor VIII exons 1 alone, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14 or 1 to 15 (for mediating 5' exon replacement in the pre-mRNA), such that the nucleic acid molecule is recognised by nuclear splicing components within the cell.

The methods of the invention encompass contacting the PTMs of the invention with a natural target human Factor VIII pre-mRNA under conditions in which a portion of the PTM is spliced to the natural FVIII pre-mRNA to correct genetic mutations found to be associated with genetic diseases, more particularly disorders of FVIII such as hemophilia A.

As indicated above, the general design, construction and genetic engineering of PTMs and demonstration of their ability to successful mediate trans-splicing reactions within the cell are described in detail in U. S. Patent Nos. 6,083,702, 6,013,487 and 6,280,978 and elsewhere. The methods thus enabled comprise contacting the PTMs of the invention with a cell expressing a FVIII target pre-mRNA under conditions in which the PTM is taken up by the cell and a portion of the synthetic PTM is trans-spliced to a portion of the target pre-mRNA to form a novel chimeric RNA molecule that results in correction of a FVIII genetic defect. Alternatively, nucleic acid molecules encoding PTMs may be delivered into a target cell followed by expression of the nucleic acid molecule to form a PTM capable of mediating a trans-splicing reaction. The PTMs of the invention are genetically engineered so that the novel chimeric RNA resulting from the trans-splicing reaction encodes a protein that complements or corrects a defective or inactive FVIII protein within the cell.

### 4. BRIEF DESCRIPTION OF THE DRAWINGS

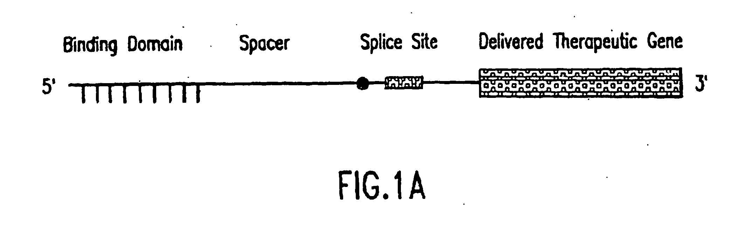

Figure 1A. Model of Pre-Trans-splicing RNA.

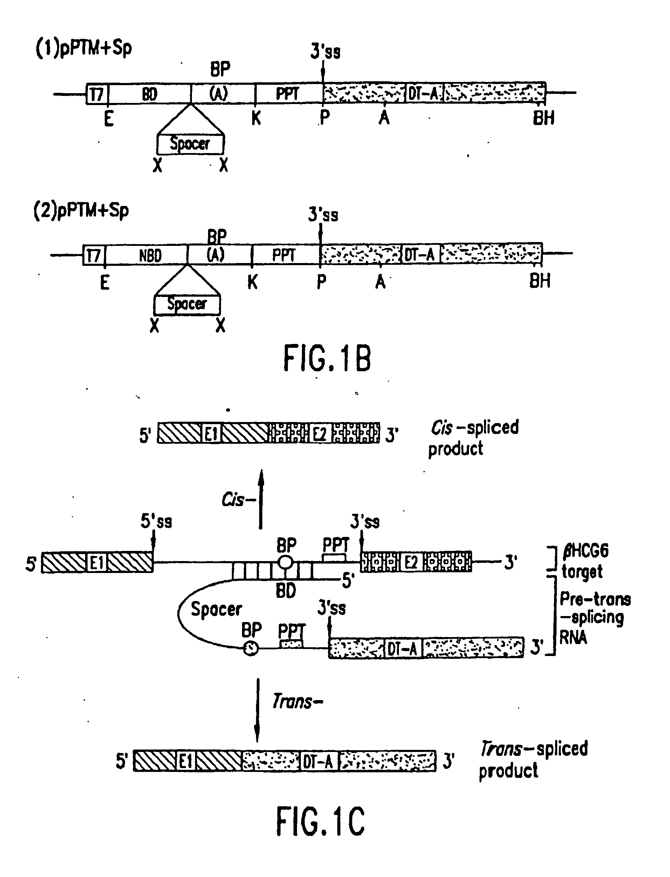

Figure 1B. Model PTM constructs and targeted trans-splicing strategy. Schematic representation of the first generation PTMs (PTM+Sp and PTM- Sp). BD, binding domain; NBD, non-binding domain; BP, branch point; PPT, pyrimidine tract; ss, splice site and DT-A, diphtheria toxin subunit A. Unique restriction sites within the PTMS are indicated by single letters: E; EcoRI ; X, Xhol; K, Kpnl; P, Pstl ; A, Accl ; B, BamHI and H; HindIII.
Figure 1C. Schematic drawing showing the binding of PTM+Sp via conventional Watson Crick base pairing to the PHCG6 target pre-mRNA and the proposed cis-and trans-splicing mechanism.
Figure 2. Schematic diagram of a LacZ-CFTR 5'exon replacement PTM showing the new design features that enhance trans-splicing efficiency. The design features include a mini intron, Tia-1 consensus binding sites and ribozyme sequences.
Figure 3. LacZ-CFTR repair model for assessing trans-splicing efficiency. A PTM is shown binding to a chimeric LacZ-CFTR mini-gene target, at the pre-mRNA level. Correctly trans-spliced target leads to the formation of a functional lacZ protein.
Figure 4. Comparison between two LacZ constructs targeted to either the 5'donor site of the target intron (PTM45) or the 3'end of the same intron (PTM52). Targeting to the 3'end of the target intron increases activity by-3 fold. Values are mean ± SE (n = 4).
Figure 5. Comparison between two LacZ constructs targeted to either the 5'donor site of the target intron (PTM50) or the 3'end of the same intron (PTM53). With this form of construct targeting to the 3' end of the target intron increases activity by ~8 fold. Values are mean ± SE (n = 3).
Figure 6. Comparison between three different LacZ constructs. The construct containing a Tia-1 element (PTM58) shows 25% higher activity compared to PTM without this element (PTM59). Constructs that contain a mini-intron in the LacZ coding (PTM58) are ~3 fold more active than those without (PTM54). Values are mean ± SE (n = 2).
Figure 7. Comparison between LacZ constructs with (PTM47 and PTM48) and without (PTM45) a cis-acting ribozyme. Values are mean ± SE (n = 3).
Figure 8. Schematic representation of different *trans*-splicing reactions. (a) *trans*-splicing reactions between the target 5' splice site and PTM's 3' splice site, (b) *trans*-splicing reactions between the target 3' splice site and PTM's 5' splice site and (c) replacement of an internal exon by a double *trans*-splicing reaction in which the PTM carries both 3' and 5' splice sites. BD, binding domain; BP, branch point sequence; PPT, polypyrimidine tract; and ss, splice sites.
Figure 9. Model system for correction of mutant murine FVIII mRNA by *trans*-splicing.

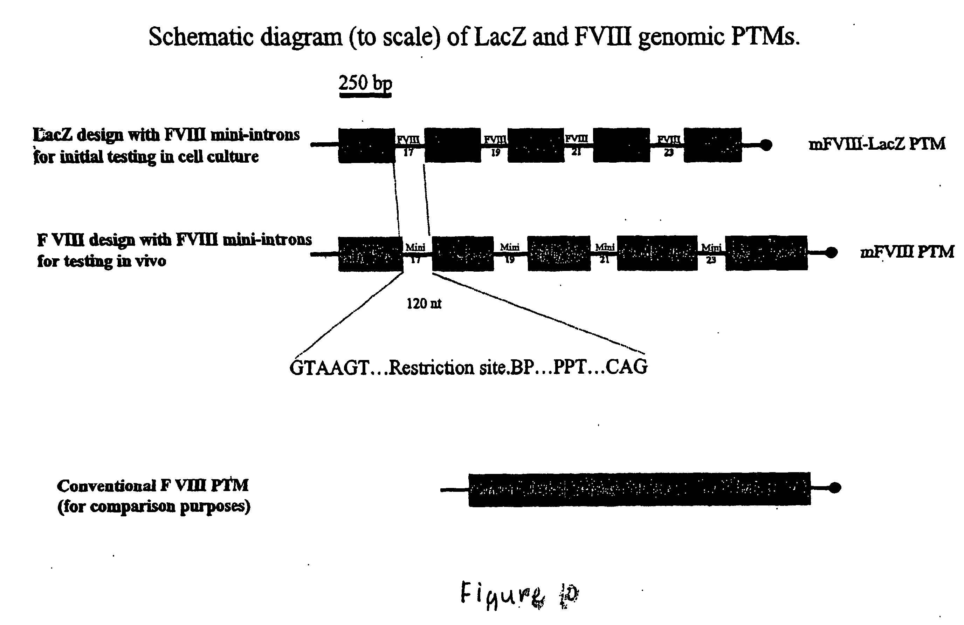

Figure 10. Schematic representation of LacZ and FVIII genomic PTMs.
Figure 11. Canine FVIII Repair Model.
Figure 12. Schematic representation of a canine FVIII PTM.

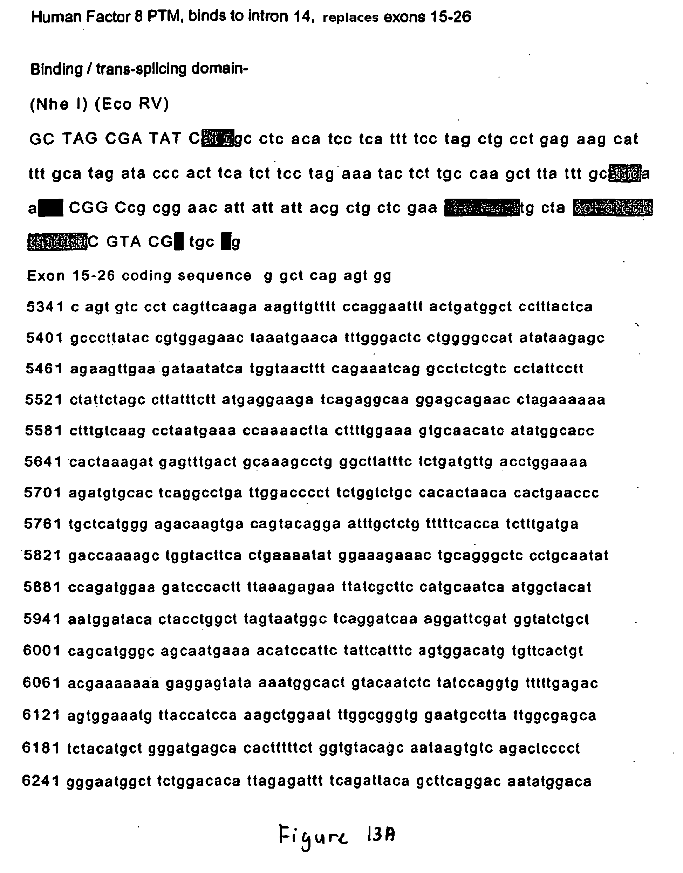

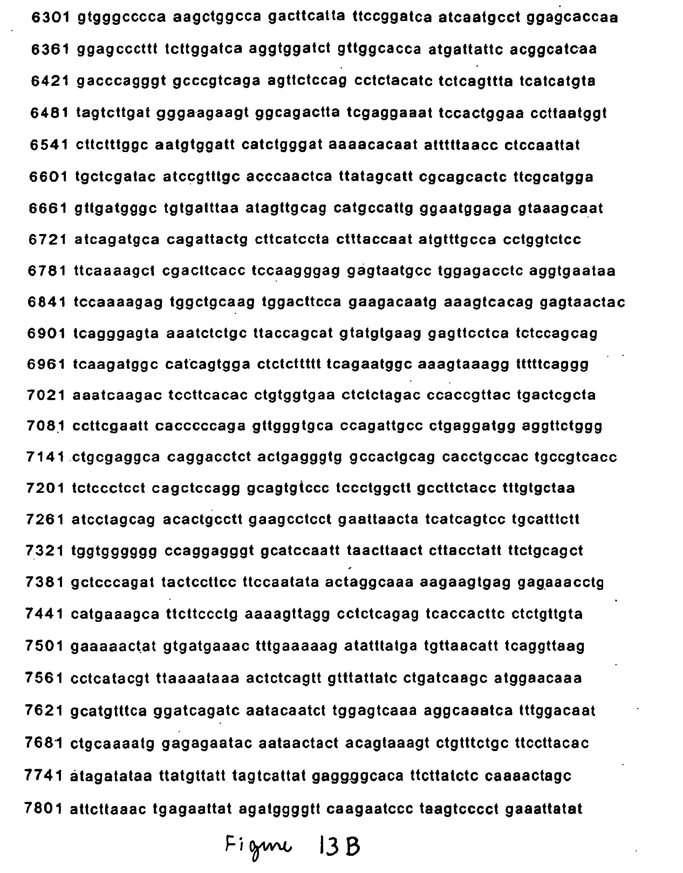

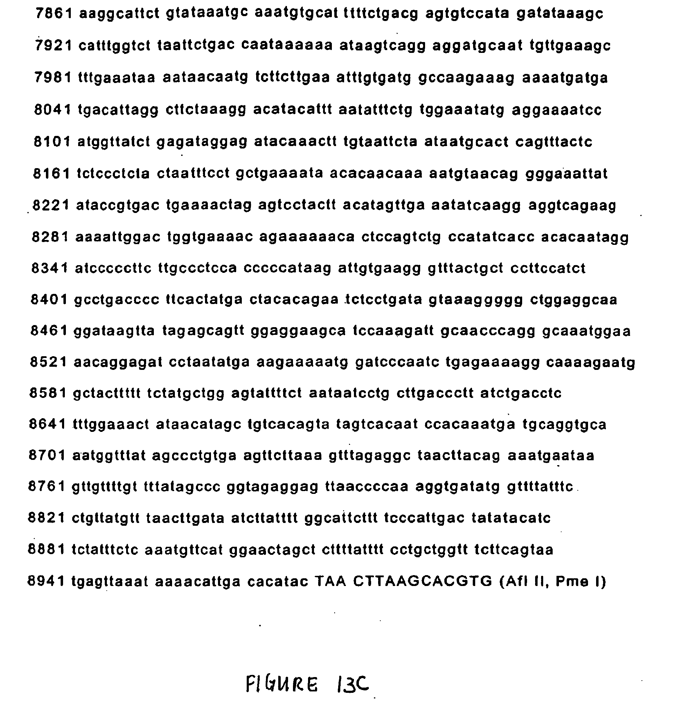

Figure 13A-C. Human FVIII PTM designed to bind to intron 14 and replace exons 15-26.
Figure 14A. Detailed structure of the mouse factor VIII PTM containing normal mouse sequences for exons 16-26. BGH=bovine growth hormone 3' UTR (untranslated sequence); Binding Domain=125bp; base changes to eliminate cryptic sites are circled:F5, F6, F7, F8=primer sites.
Figure 14B. Schematic diagram showing the extent of the binding domain in the mouse factor VIII gene.
Figure 14C. Changes to the promoter in AAV vectors pDLZ20 and pDLZ20-M2 to eliminate cryptic donor sites in sequence upstream of the murine PTM binding domain.
Figure 14D. Murine Factor VIII repair model. Schematic diagram of a PTM binding to the 3' splice site of intron 15 of the mouse Factor VIII gene.

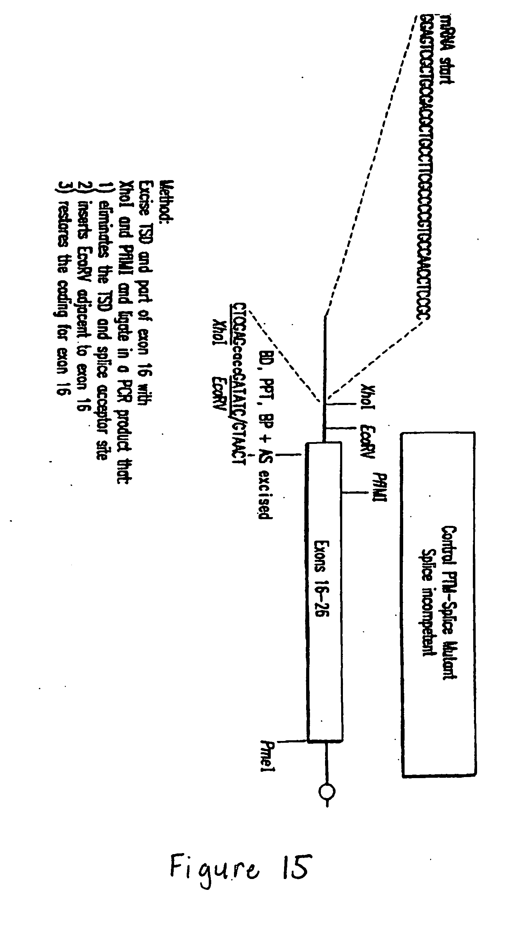

Figure 15. Schematic diagram of a F8 PTM with the trans-splicing domain eliminated. This represents a control PTM to test whether repair is a result of trans-splicing or complementation at the protein level.
Figure 16. Data indicating repair of Factor VIII in Factor VIII knock out mice. Blood was assayed for factor VIII activity using a coatest assay.

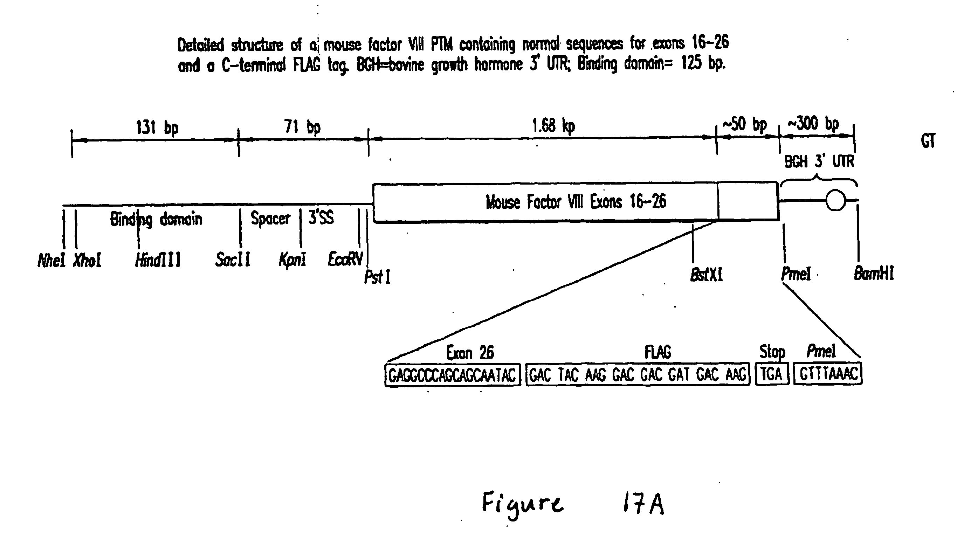

Figure 17A. Detailed structure of a mouse Factor VIII PTM containing normal sequences for exons 16-26 and a C-terminal FLAG tag. BGH=bovine growth hormone 3"UTR; Binding domain=125 bp.

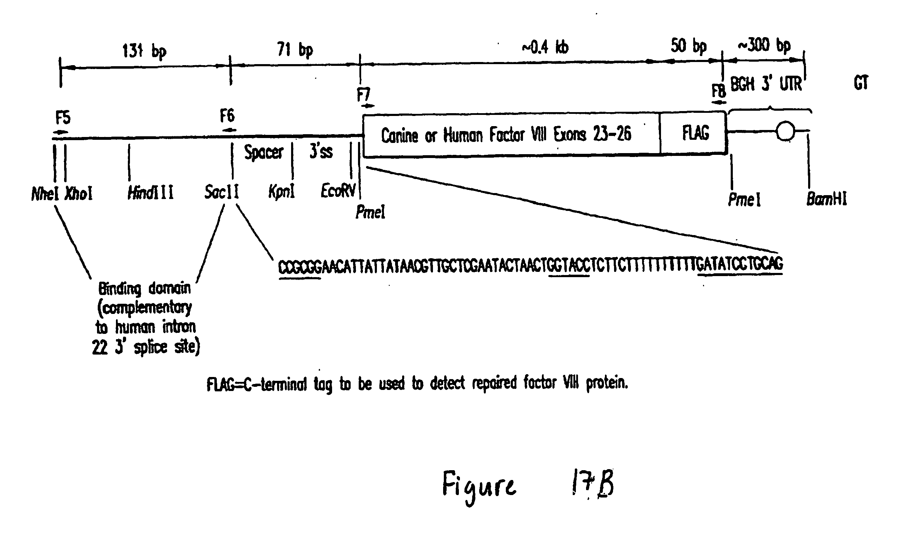

Figure 17B. Detailed structure of a human or canine Factor VIII PTM containing normal sequences for exons 23-26.

### 5. DETAILED DESCRIPTION OF THE INVENTION

The present invention relates to novel compositions comprising pre- trans-splicing molecules (PTMs) and the use of such molecules for generating novel nucleic acid molecules. The PTMs of the invention comprise (i) one or more target binding domains that are designed to specifically bind to pre-mRNA, (ii) a 3' splice region that includes a branch point, pyrimidine tract and a 3' splice acceptor site and/or a 5' splice donor site (iii) one or more spacer regions that separate the RNA splice site from the target binding domain and (iv) a nucleotide sequence of (1) human Factor VIII exons 15 to 26, 16 to 26, 17 to 26, 18 to 26, 19 to 26, 20 to 26, 21 to 26 or 22 to 26, to be trans-spliced to the target pre-mRNA (for mediating 3' exon replacement in the pre-mRNA), or (2) human Factor VIII exons 1 alone, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14 or 1 to 15 (for mediating 5' exon replacement in the pre-mRNA). Such a PTM may further comprise (a) mini introns, (b) ISAR (intronic splicing activator and repressor) consensus binding sites, and/or (c) ribozyme sequences.

As hereinbefore indicated, there is thus enabled contacting the PTMs of the invention with a natural human Factor VIII pre-mRNA under conditions in which a portion of the PTM is trans-spliced to a portion of the natural pre-mRNA whereby there is correction of a FVIII genetic defect.

### 5.1. STRUCTURE OF THE PRE-TRANS-SPLICING MOLECULES

The PTMs of the invention, like previously described PTMs for targeted trans-splicing, comprise (i) one or more target binding domains that target binding of the PTM to a pre-mRNA (ii) a 3' splice region that includes a branch point, pyrimidine tract and a 3' splice acceptor site and/or 5' splice donor site; and (iii) may also include at least one of the following features: (a) binding domains targeted to intron sequences in close proximity to the 3' or 5' splice signals of the target intron, (b) mini introns, (c) ISAR (intronic splicing activator and repressor) consensus binding sites, and/or (d) ribozyme sequences. The PTMs of the invention include one or more spacer regions to separate the RNA splice site from the target binding domain. Additionally, the PTMs are engineered to contain any nucleotide sequence encoding a portion of human Factor VIII as hereinbefore indicated.

The target binding domain of the PTM endows the PTM with a binding affinity for the target pre-mRNA. As used herein, a target binding domain is defined as any molecule, i.e., nucleotide, protein, chemical compound, etc. , that confers specificity of binding and anchors the pre-mRNA closely in space to the synthetic PTM so that the spliceosome processing machinery of the nucleus can trans-splice a portion of the synthetic PTM to a portion of the pre-mRNA.

The target binding domain of the PTM may contain multiple binding domains which are complementary to and in anti-sense orientation to the targeted region of the selected pre-mRNA. The target binding domains may comprise up to several thousand nucleotides. In preferred embodiments of the invention the binding domains may comprise at least 10 to 30 and up to several hundred or more nucleotides. The specificity of the PTM may be increased significantly by increasing the length of the target binding domain. For example, the target binding domain may comprise several hundred nucleotides or more. In addition, although the target binding domain may be "linear" it is understood that the RNA will very likely fold to form secondary structures that may stabilize the complex thereby increasing the efficiency of splicing. A second target binding region may be placed at the 3' end of the molecule and can be incorporated into the PTM of the invention. Absolute complementarily, although preferred, is not required. A sequence "complementary" to a portion of an RNA, as referred to herein, means a sequence having sufficient complementarity to be able to hybridize with the target pre-mRNA, forming a stable duplex. The ability to hybridize will depend on both the degree of complementarity and the length of the nucleic acid (See, for example, Sambrook et al., 1989, Molecular Cloning, A Laboratory Manual, 2d Ed., Cold Spring Harbor Laboratory Press, Cold Spring Harbor, New York). Generally, the longer the hybridizing nucleic acid, the more base mismatches with an RNA it may contain and still form a stable duplex. One skilled in the art can ascertain a tolerable degree of mismatch or length of duplex by use of standard procedures to determine the stability of the hybridized complex.

Binding may also be achieved through other mechanisms, for example, through triple helix formation, aptamer interactions, antibody interactions or protein/nucleic acid interactions such as those in which the PTM is engineered to recognize a specific RNA binding protein, *i.e*., a protein bound to a specific target pre-mRNA. Alternatively, the PTMs of the invention may be designed to recognize secondary structures, such as for example, hairpin structures resulting from intramolecular base pairing between nucleotides within an RNA molecule.

In a specific embodiment of the invention, the binding domain of the 5' exon replacement PTM is targeted to bind to intron sequences in close proximity to the 3' splice signals of the intron. Targeting of the PTM to the 3' end of the intron is intended to bring the PTM donor site in close proximity to the target acceptor site. In embodiments of the invention the PTM binding site is targeted to bind between 20 and several thousand nucleotides from the 3' intron sequences.

In a specific embodiment of the invention, the target binding domain is complementary and in anti-sense orientation to sequences in close proximity to the region of the FVIII target pre-mRNA targeted for *trans*-splicing. For example, a target binding domain may be defined as any molecule, *i.e*., nucleotide, protein, chemical compound, etc., that confers specificity of binding and anchors the FVIII pre-mRNA closely in space to the PTM so that the spliceosome processing machinery of the nucleus can trans-splice a portion of the PTM to a portion of the FVIII pre- mRNA.

The PTM molecule also contains a 3' splice region that includes a branchpoint sequence and a 3' splice acceptor AG site and/or a 5' splice donor site. The 3' splice region further comprises a polypyrimidine tract. Consensus sequences for the 5' splice donor site and the 3' splice region used in RNA splicing are well known in the art (See, Moore, et al., 1993, The RNA World, Cold Spring Harbor Laboratory Press, p. 303-358). In addition, modified consensus sequences that maintain the ability to function as 5' donor splice sites and 3' splice regions may be used in the practice of the invention. Briefly, the 5' splice site consensus sequence is AG/GURAGU (where A=adenosine, U=uracil, G=guanine, C=cytosine, R=purine and/=the splice site). The 3' splice site consists of three separate sequence elements: the branchpoint or branch site, a polypyrimidine tract and the 3' consensus sequence (YAG). The branch point consensus sequence in mammals is YNYURAC (Y=pyrimidine; N=any nucleotide). The underlined A is the site of branch formation. A polypyrimidine tract is located between the branch point and the splice site acceptor and is important for different branch point utilization and 3' splice site recognition. Recently, pre-mRNA introns beginning with the dinucleotide AU and ending with the dinucleotide AC have been identified and referred to as U12 introns. U12 intron sequences as well as any sequences that function as splice acceptor/donor sequences may also be used to generate the PTMs of the invention.

A spacer region to separate the RNA splice site from the target binding domain will be included in the PTM. The spacer region may be designed to include features such as stop codons which would block any translation of an unspliced PTM and/or sequences that enhance trans-splicing to the target pre-mRNA.

In a preferred embodiment of the invention, a "safety" is also incorporated into the spacer, binding domain, or elsewhere in the PTM to prevent non-specific trans-splicing. This is a region of the PTM that covers elements of the 3' and/or 5' splice site of the PTM by relatively weak complementarity, preventing non- specific trans-splicing. The PTM is designed in such a way that upon hybridization of the binding/targeting portion (s) of the PTM, the 3' and/or 5' splice site is uncovered and becomes fully active.

The "safety" consists of one or more complementary stretches of cis-sequence (or could be a second, separate, strand of nucleic acid) which binds to one or both sides of the PTM branch point, pyrimidine tract, 3' splice site and/or 5' splice site (splicing elements), or could bind to parts of the splicing elements themselves. This "safety" binding prevents the splicing elements from being active (i. e. block U2 snRNP or other splicing factors from attaching to the PTM splice site recognition elements). The binding of the "safety" may be disrupted by the binding of the target binding region of the PTM to the target pre-mRNA, thus exposing and activating the PTM splicing elements (making them available to trans-splice into the target pre- mRNA).

Additionally, the PTMs of the invention contain FVIII exon sequences designed to correct a FVIII genetic defect. A variety of different PTM molecules may be synthesized for use in the production of a novel chimeric RNA which complements a defective or inactive FVIII protein. The PTMs of the invention may contain FVIII exon sequences, which when trans-spliced to the FVIII target pre- mRNA, will result in the formation of a composite or chimeric RNA capable of encoding a functional FVIII protein. The nucleotide sequence of the FVIII gene is known and incorporated herein in its entirety (NCBI Accession Nos. M88628- M88648; see also Truett et al., 1985, DNA 4: 333-349; http ://europium. csc. mrc. ac.uk/usr/WWW/WebPages /main. dir/main. htm).

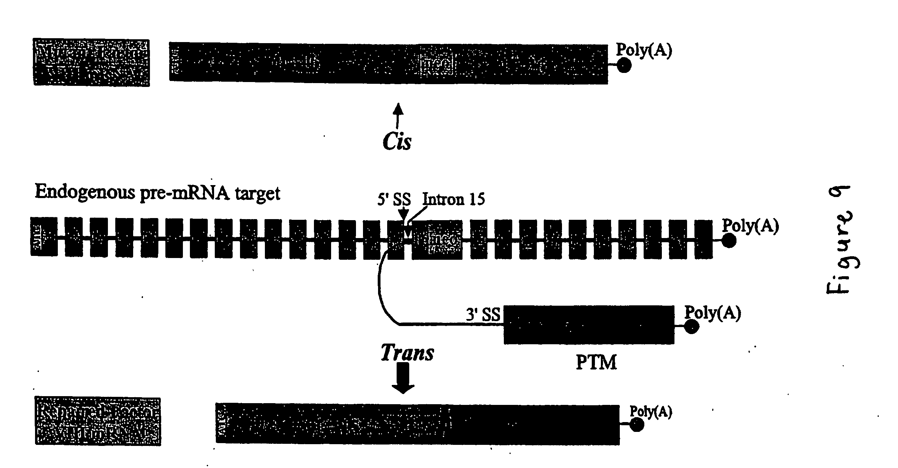

The FVIII exon sequences to be included in the structure of the PTM will depend on the specific FVIIII mutation targeted for correction. For example, when targeting correction of a mutation in FVIII exon 16, the PTM will be designed to include FVIII exons 16-26 sequences as depicted in Figure 9. In such an instance, 3'exon replacement will result in the formation of a chimeric RNA molecule that encodes for a functional FVIII protein. The PTM's of the invention may be engineered to contain a single FVIII exon sequence or multiple FVIII exon sequences. The number and identity of the FVIII sequences to be used in the PTMs will depend on the targeted FVIII mutation, and the type of trans-splicing reaction, i. e., 5'exon replacement, 3'exon replacement or internal exon replacement that will occur.

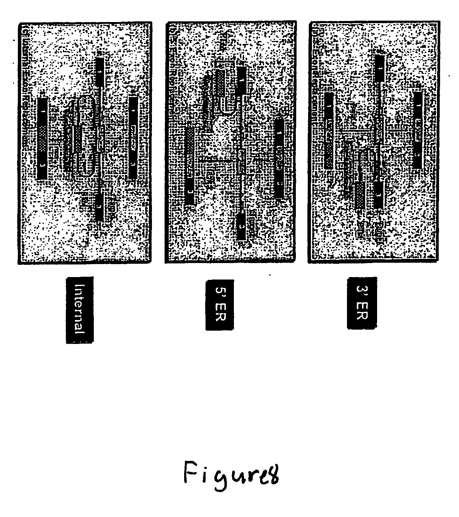

Specific PTMs of the invention, include but are not limited to, those containing nucleic acids encoding FVIII exons 15-26,16-26, 17-26,18-26, 19-26, 20-26,21-26 or 22-26. Such PTMs may be used for mediating a 3'exon replacement trans-splicing reaction as depicted in Figure 8.

Specific PTMs of the invention, include but are not limited to, those containing nucleic acids encoding FVIII exon 1 or exons 1-2,1-3, 1-4,1-5, 1-6,1-7, 1-8,1-9, 1-10,1-11, 1-12,1-13, 1-14 or 1-15. Such PTMs may be used for mediating a 5'exon replacement trans-splicing reaction as depicted in Figure 8.

Thus, PTMs of the invention may comprise a single FVIII exon or combination of two or more FVIII exons.

In addition, to limit the size of the PTM, the molecule may include deletions in non-essential regions of the FVIII gene. For example, deletions of the B domain encoded by exon 14, have been found to retain biological activity.

The present invention further provides PTM molecules wherein the coding region of the PTM is engineered to contain mini-introns. The insertion of mini-introns into the coding sequence of the PTM is designed to increase definition of the exon and enhance recognition of the PTM donor site. Mini-intron sequences to be inserted into the coding regions of the PTM include small naturally occurring introns or, alternatively, any intron sequences, including synthetic mini-introns, which include 5' consensus donor sites and 3' consensus sequences which include a branch point, a 3' splice site and in some instances a pyrimidine tract.

The mini-intron sequences are preferably between about 60-150 nucleotides in length, however, mini-intron sequences of increased lengths may also be used. In a preferred embodiment of the invention, the mini-intron comprises the 5' and 3' end of an endogenous intron. In preferred embodiments of the invention the 5' intron fragment is about 20 nucleotides in length and the 3' end is about 40 nucleotides in length.

In a specific embodiment of the invention, an intron of 528 nucleotides comprising the following sequences may be utilized. Sequence of the intron construct is as follows:
5' fragment sequence: 
Gtagttcttttgttcttcactattaagaacttaatttggtgtccatgtctcttttttttctagtttgtagtgctgg
aaggtatttttggagaaattcttacatgagcattaggagaatgtatgggtgtagtgtcttgtataatagaaattgttccactgata
atttactctagtttttatttcctcatattatttcagtggctttttcttccacatctttatatttgcaccacattcaacactgtagcggc
cgc.
3' fragment sequence:

Ccaactatctgaatcatgtgccccttctctgtgaacctctatcataatacttgtcacactgtattgtaattg
tctcttttactttcccttgtatcttttgtgcatagcagagtacctgaaacaggaagtattttaaatattttgaatcaaatgagttaata
gaatctttacaaataagaatatacacttctgcttaggatgataattggaggcaagtgaatcctgagcgtgatttgataatgacct
aataatgatgggttttatttccag

In yet another specific embodiment of the invention, consensus ISAR sequences are includes in the PTMs of the invention (Jones et al., NAR 29:3557-3565). Proteins bind to the ISAR splicing activator and repressor consensus sequence which includes a uridine-rich region that is required for 5' splice site recognition by U1 SnRNP. The 18 nucleotide ISAR consensus sequence comprises the following sequence: GGGCUGAUUUUUCCAUGU. When inserted into the PTMs of the invention, the ISAR consensus sequences are inserted into the structure of the PTM in close proximity to the 5' donor site of intron sequences. In an embodiment of the invention the ISAR sequences are inserted within 100 nucleotides from the 5' donor site. In a preferred embodiment of the invention the ISAR sequences are inserted within 50 nucleotides from the 5' donor site. In a more preferred embodiment of the invention the ISAR sequences are inserted within 20 nucleotides of the 5' donor site.

The compositions of the invention further comprise PTMs that have been engineered to include *cis*-acting ribozyme sequences. The inclusion of such sequences is designed to reduce PTM translation in the absence of *trans*-splicing or to produce a PTM with a specific length or defined end(s). The ribozyme sequences that may be inserted into the PTMs include any sequences that are capable of mediating a *cis*-acting (self-cleaving) RNA splicing reaction. Such ribozymes include but are not limited to hammerhead, hairpin and hepatitis delta virus ribozymes (see, Chow et al. 1994, J Biol Chem 269:25856-64).

In an embodiment of the invention, splicing enhancers such as, for example, sequences referred to as exonic splicing enhancers may also be included in the structure of the synthetic PTMs. Transacting splicing factors, namely the serine/arginine-rich (SR) proteins, have been shown to interact with such exonic splicing enhancers and modulate splicing (*See,* Tacke et al., 1999, Curr. Opin. Cell Biol. 11:358-362; Tian et al., 2001, J. Biological Chemistry 276:33833-33839; Fu, 1995, RNA 1:663-680). Nuclear localization signals may also be included in the PTM molecule (Dingwell and Laskey, 1986, Ann. Rev. Cell Biol. 2:367-390; Dingwell and Laskey, 1991, Trends in Biochem. Sci. 16:478-481). Such nuclear localization signals can be used to enhance the transport of synthetic PTMs into the nucleus where *trans*-splicing occurs.

Additional features can be added to the PTM molecule either after, or before, the nucleotide sequence encoding a translatable protein, such as polyadenylation signals to modify RNA expression/stability, or 5' splice sequences to enhance splicing, additional binding regions, "safety"-self complementary regions, additional splice sites, or protective groups to modulate the stability of the molecule and prevent degradation. In addition, stop codons may be included in the PTM structure to prevent translation of unspliced PTMs. Further elements such as a 3' hairpin structure, circularized RNA, nucleotide base modi fication, or synthetic analogs can be incorporated into PTMs to promote or facilitate nuclear localization and spliceosomal incorporation, and intra-cellular stability.

PTMs may also be generated that require a double-*trans*-splicing reaction for generation of a chimeric *trans*-spliced product. Such PTMs could, for example, be used to replace an internal exon which could be used for FVIII gene repair. PTMs designed to promote two *trans*-splicing reactions are engineered as described above, however, they contain both 5' donor sites and 3' splice acceptor sites. In addition, the PTMs may comprise two or more binding domains and splicer regions. The splicer regions may be placed between the multiple binding domains and splice sites or alternatively between the multiple binding domains.

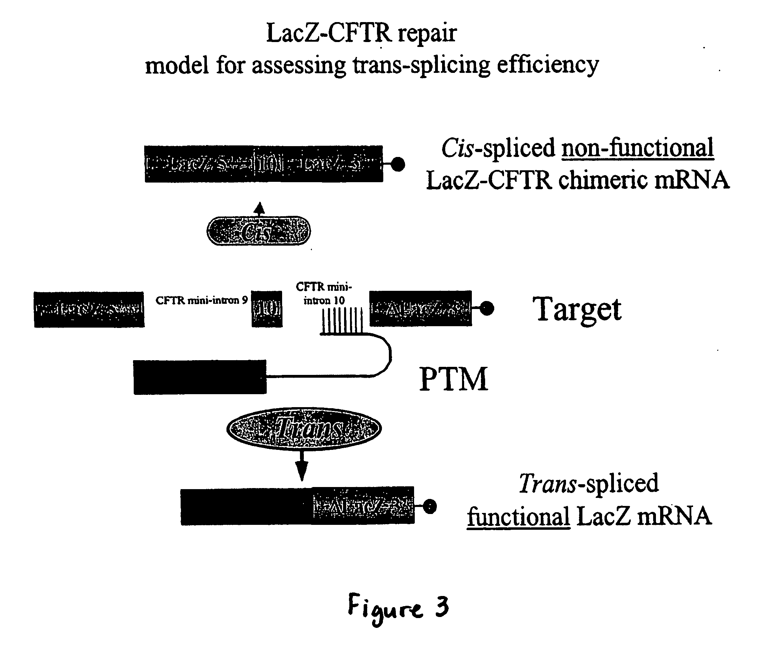

A novel lacZ based assay has been developed for identifying optimal PTM sequences for mediating a desired *trans*-splicing reaction (Figure 3). The assay permits very rapid and easy testing of many PTMs for their ability to *trans*-splice. The LacZ FVIII chimeric target is presented in Figure 3. This target consists of the coding region for LacZ (minus 120 nucleotide from the central coding region), split into a 5' "exon" and a 3' "exon". Separating these exons is a genomic fragment of the factor VIII gene of mouse including intron 15, exon 16 and intron 17. All donor and acceptor sites in this target are functional but a cis-spliced target, which generates a LacZ-FVIII chimeric mRNA, is non-functional with respect to β-gal activity. *Trans-*splicing between the PTM and target will generate a full length functional LacZ mRNA.

Each new PTM to be tested is transiently co-transfected with the LacZ-FVIII target using Lipofectamine reagents and then assayed for ß-galactocidase activity after 48 hours. Total RNA samples may also be prepared and assessed by RT-PCR using target and PTM specific primers for the presence of correctly spliced repaired products and the level of repaired product. Each *trans*-splicing domain (TSD) and binding domain is engineered with several unique restriction sites, so that when a suitable sequence is identified (based on the level of ß-galactocidase activity and RT-PCR data), part of or the complete TSD, can be readily subcloned into a factor FVIII PTM.

When specific PTMs are to be synthesized *in vitro* (synthetic PTMs), such PTMs can be modified at the base moiety, sugar moiety, or phosphate backbone, for example, to improve stability of the molecule, hybridization to the target FVIII mRNA, transport into the cell, etc. For example, modification of a PTM to reduce the overall charge can enhance the cellular uptake of the molecule. In addition modifications can be made to reduce susceptibility to nuclease or chemical degradation. The nucleic acid molecules may be synthesized in such a way as to be conjugated to another molecule such as a peptides (e.g., for targeting host cell receptors *in vivo*), or an agent facilitating transport across the cell membrane (see, *e.g.,* Letsinger et al., 1989, Proc. Natl. Acad. Sci. USA 86:6553-6556; Lemaitre et al., 1987, Proc. Natl. Acad. Sci. 84:648-652; PCT Publication No. W088/09810, published December 15, 1988) or the blood-brain barrier (see, *e.g*., PCT Publication No. W089/10134, published April 25, 1988), hybridization-triggered cleavage agents (see, *e.g*., Krol et al., 1988, BioTechniques 6:958-976) or intercalating agents (see, *e.g*., Zon, 1988, Pharm. Res. 5:539-549). To this end, the nucleic acid molecules may be conjugated to another molecule, *e.g*., a peptide, hybridization triggered cross-linking agent, transport agent, hybridization-triggered cleavage agent, etc.

Various other well-known modifications to the nucleic acid molecules can be introduced as a means of increasing intracellular stability and half-life. Possible modifications include, but are not limited to, the addition of flanking sequences of ribonucleotides to the 5' and/or 3' ends of the molecule. In some circumstances where increased stability is desired, nucleic acids having modified internucleoside linkages such as 2'-0-methylation may be preferred. Nucleic acids containing modified internucleoside linkages may be synthesized using reagents and methods that are well known in the art (see, Uhlmann et al., 1990, Chem. Rev. 90:543-584; Schneider et al., 1990, Tetrahedron Lett. 31:335 and references cited therein).

The synthetic PTMs of the present invention are preferably modified in such a way as to increase their stability in the cells. Since RNA molecules are sensitive to cleavage by cellular ribonucleases, it may be preferable to use as the competitive inhibitor a chemically modified oligonucleotide (or combination of oligonucleotides) that mimics the action of the RNA binding sequence but is less sensitive to nuclease cleavage. In addition, the synthetic PTMs can be produced as nuclease resistant circular molecules with enhanced stability to prevent degradation by nucleases (Puttaraju et al., 1995, Nucleic Acids Symposium Series No. 33:49-51; Puttaraju et al., 1993, Nucleic Acid Research 21:4253-4258). Other modifications may also be required, for example to enhance binding, to enhance cellular uptake, to improve pharmacology or pharmacokinetics or to improve other pharmaceutically desirable characteristics.

Modifications, which may be made to the structure of the synthetic PTMs include but are not limited to backbone modifications such as use of:
(i) phosphorothioates (X or Y or W or Z=S or any combination of two or more with the remainder as O). *e.g*. Y=S (Stein, C. A., et al., 1988, Nucleic Acids Res., 16:3209-3221), X=S (Cosstick, R., et al., 1989, Tetrahedron Letters, 30, 4693-4696), Y and Z=S (Brill, W. K.-D., et al., 1989, J. Amer. Chem. Soc., 111:2321-2322); (ii) methylphosphonates (*e.g.* Z=methyl (Miller, P. S., et al., 1980, J. Biol. Chem., 255:9659-9665); (iii) phosphoramidates (Z=N-(alkyl)₂ *e.g.* alkyl methyl, ethyl, butyl) (Z=morpholine or piperazine) (Agrawal, S., et al., 1988, Proc. Natl. Acad. Sci. USA 85:7079-7083) (X or W=NH) (Mag, M., et al., 1988, Nucleic Acids Res., 16:3525-3543); (iv) phosphotriesters (Z=O-alkyl *e.g*. methyl, ethyl, *etc*) (Miller, P. S., et al., 1982, Biochemistry, 21:5468-5474); and (v) phosphorus-free linkages (*e.g*. carbamate, acetamidate, acetate) (Gait, M. J., et al., 1974, J. Chem. Soc. Perkin I, 1684-1686; Gait, M. J., et al., 1979, J. Chem. Soc. Perkin I, 1389-1394).

In addition, sugar modifications may be incorporated into the PTMs of the invention. Such modifications include the use of: (i) 2'-ribonucleosides (R=H); (ii) 2'-O-methylated nucleosides (R=OMe)) (Sproat, B. S., et al., 1989, Nucleic Acids Res., 17:3373-3386); and (iii) 2'-fluoro-2'-riboxynucleosides (R=F) (Krug, A., et al., 1989, Nucleosides and Nucleotides, 8:1473-1483).

Further, base modifications that may be made to the PTMs, including but not limited to use of: (i) pyrimidine derivatives substituted in the 5-position (e.g. methyl, bromo, fluoro etc) or replacing a carbonyl group by an amino group (Piccirilli, J. A., et al., 1990, Nature, 343:33-37); (ii) purine derivatives lacking specific nitrogen atoms (*e.g*. 7-deaza adenine, hypoxanthine) or functionalized in the 8-position (*e.g*. 8-azido adenine, 8-bromo adenine) (for a review see Jones, A. S., 1979, Int. J. Biolog. Macromolecules,1:194-207).

In addition, the PTMs may be covalently linked to reactive functional groups, such as: (i) psoralens (Miller, P. S., et al., 1988, Nucleic Acids Res., Special Pub. No. 20, 113-114), phenanthrolines (Sun, J-S., et al., 1988, Biochemistry, 27:6039-6045), mustards (Vlassov, V. V., et al., 1988, Gene, 72:313-322) (irreversible cross-linking agents with or without the need for co-reagents); (ii) acridine (intercalating agents) (Helene, C., et al., 1985, Biochimie, 67:777-783); (iii) thiol derivatives (reversible disulphide formation with proteins) (Connolly, B. A., and Newman, P. C., 1989, Nucleic Acids Res., 17:4957-4974); (iv) aldehydes (Schiffs base formation); (v) azido, bromo groups (UV cross-linking); or (vi) ellipticines (photolytic cross-linking) (Perrouault, L., et al., 1990, Nature, 344:358-360).

In an embodiment of the invention, oligonucleotide mimetics in which the sugar and internucleoside linkage, *i.e*., the backbone of the nucleotide units, are replaced with novel groups can be used. For example, one such oligonucleotide mimetic which has been shown to bind with a higher affinity to DNA and RNA than natural oligonucleotides is referred to as a peptide nucleic acid (PNA) (for review see, Uhlmann, E. 1998, Biol. Chem. 379:1045-52). Thus, PNA may be incorporated into synthetic PTMs to increase their stability and/or binding affinity for the target pre-mRNA.

In another embodiment of the invention synthetic PTMs may covalently linked to lipophilic groups or other reagents capable of improving uptake by cells. For example, the PTM molecules may be covalently linked to: (i) cholesterol (Letsinger, R. L., et al., 1989, Proc. Natl. Acad. Sci. USA, 86:6553-6556); (ii) polyamines (Lemaitre, M., et al., 1987, Proc. Natl. Acad. Sci, USA, 84:648-652); other soluble polymers (*e.g*. polyethylene glycol) to improve the efficiently with which the PTMs are delivered to a cell. In addition, combinations of the above identified modifications may be utilized to increase the stability and delivery of PTMs into the target cell. The PTMs of the invention can be used in methods designed to produce a novel chimeric RNA in a target cell. The methods of the present invention comprise delivering to the target cell a PTM which may be in any form used by one skilled in the art, for example, an RNA molecule, or a DNA vector which is transcribed into a RNA molecule, wherein said PTM binds to a pre-mRNA and mediates a *trans*-splicing reaction resulting in formation of a chimeric RNA comprising a portion of the PTM molecule spliced to a portion of the pre-mRNA.

In a specific embodiment of the invention, the PTMs of the invention can be used in methods designed to produce a novel chimeric RNA in a target cell so as to result in correction of clotting FVIII clotting defects. The methods of the present invention comprise delivering to a cell a PTM which may be in any form used by one skilled in the art, for example, an RNA molecule, or a DNA vector which is transcribed into a RNA molecule, wherein said PTM binds to a FVIII pre-mRNA and mediates a *trans*-splicing reaction resulting in formation of a chimeric RNA comprising a portion of the PTM molecule spliced to a portion of the pre-mRNA.

### 5.2. SYNTHESIS OF THE TRANS-SPLICING MOLECULES

The nucleic acid molecules of the invention can be RNA or DNA or derivatives or modified versions thereof, single-stranded or double-stranded. By nucleic acid is meant a PTM molecule or a nucleic acid molecule encoding a PTM molecule, whether composed of deoxyribonucleotides or ribonucleosides, and whether composed of phosphodiester linkages or modified linkages. The term nucleic acid also specifically includes nucleic acids composed of bases other than the five biologically occurring bases (adenine, guanine, thymine, cytosine and uracil). In addition, the PTMs of the invention may comprise, DNA/RNA, RNA/protein or DNA/RNA/protein chimeric molecules that are designed to enhance the stability of the PTMs.

The PTMs of the invention can be prepared by any method known in the art for the synthesis of nucleic acid molecules. For example, the nucleic acids may be chemically synthesized using commercially available reagents and synthesizers by methods that are well known in the art (see, e.g., Gait, 1985, Oligonucleotide Synthesis: A Practical Approach, IRL Press, Oxford, England).

Alternatively, synthetic PTMs can be generated by *in vitro* transcription of DNA sequences encoding the PTM of interest. Such DNA sequences can be incorporated into a wide variety of vectors downstream from suitable RNA polymerase promoters such as the T7, SP6, or T3 polymerase promoters. Consensus RNA polymerase promoter sequences include the following:
T7: TAATACGACTCACTATAGGGAGA
SP6: ATTTAGGTGACACTATAGAAGNG
T3: AATTAACCCTCACTAAAGGGAGA.

The base in bold is the first base incorporated into RNA during transcription. The underline indicates the minimum sequence required for efficient transcription.

RNAs may be produced in high yield via *in vitro* transcription using plasmids such as SPS65 and Bluescript (Promega Corporation, Madison, WI). In addition, RNA amplification methods such as Q-β amplification can be utilized to produce the PTM of interest.

The PTMs may be purified by any suitable means, as are well known in the art. For example, the PTMs can be purified by gel filtration, affinity or antibody interactions, reverse phase chromatography or gel electrophoresis. Of course, the skilled artisan will recognize that the method of purification will depend in part on the size, charge and shape of the nucleic acid to be purified.

The PTM's of the invention, whether synthesized chemically, *in vitro,* or *in vivo,* can be synthesized in the presence of modified or substituted nucleotides to increase stability, uptake or binding of the PTM to a target pre-mRNA. In addition, following synthesis of the PTM, the PTMs may be modified with peptides, chemical agents, antibodies, or nucleic acid molecules, for example, to enhance the physical properties of the PTM molecules. Such modifications are well known to those of skill in the art.

In instances where a nucleic acid molecule encoding a PTM is utilized, cloning techniques known in the art may be used for cloning of the nucleic acid molecule into an expression vector. Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

The DNA encoding the PTM of interest may be recombinantly engineered into a variety of host vector systems that also provide for replication of the DNA in large scale and contain the necessary elements for directing the transcription of the PTM. The use of such a construct to transfect target cells in the patient will result in the transcription of sufficient amounts of PTMs that will form complementary base pairs with the endogenously expressed FVIII pre-mRNA target and thereby facilitate a trans-splicing reaction between the complexed nucleic acid molecules. For example, a vector can be introduced in vivo such that it is taken up by a cell and directs the transcription of the PTM molecule. Such a vector can remain episomal or become chromosomally integrated, as long as it can be transcribed to produce the desired RNA, i. e. PTM. Such vectors can be constructed by recombinant DNA technology methods standard in the art.

Vectors encoding the PTM of interest can be plasmids, viral, or others known in the art, used for replication and expression in mammalian cells. Expression of the sequence encoding the PTM can be regulated by any promoter/enhancer sequences known in the art to act in mammalian, preferably human cells. Such promoters/enhancers can be inducible or constitutive. Such promoters include but are not limited to: the SV40 early promoter region (Benoist, C. and Chambon, P. 1981, Nature 290: 304-310), the promoter contained in the 3'long terminal repeat of Rous sarcoma virus (Yamamoto et al., 1980, Cell 22 : 787-797), the herpes thymidine kinase promoter (Wagner et al., 1981, Proc. Natl. Acad. Sci. U. S. A. 78: 14411445), the regulatory sequences of the metallothionein gene (Brinster et al., 1982, Nature 296: 39-42), the viral CMV promoter, the human chorionic gonadotropin-P promoter (Hollenberg et al., 1994, Mol. Cell. Endocrinology 106: 111-119), etc.

In a specific embodiment of the invention, a liver specific promoter/enhancer sequences may be used to promote the synthesis of PTMs in liver cells for correction of a FVIII defect. Such promoters include, for example, the albumin, transthyretin CMV enhancers/chicken beta-actin promoter, ApoE enhancer alphal-antitrypsin promoter and endogenous FVIII promoter elements. In addition, the liver-specific microglobulin promoter cassette optimized for FVIII gene expression may be used, as well as, post-transcriptional elements such as the wood chuck post-transcriptional regulatory element (WPRE).

Any type of plasmid, cosmid, YAC or viral vector can be used to prepare the recombinant DNA construct which can be introduced directly into the tissue site. Alternatively, viral vectors can be used which selectively infect the desired target cell. Vectors for use in the practice of the invention include any eukaryotic expression vectors, including but not limited to viral expression vectors such as those derived from the class of retroviruses, adenoviruses or adeno-associated viruses.

A number of selection systems can also be used, including but not limited to selection for expression of the herpes simplex virus thymidine kinase, hypoxanthine-guanine phosphoribosyltransterase and adenine phosphoribosyl transferase protein in tk-, hgprt-or aprt-deficient cells, respectively. Also, anti- metabolic resistance can be used as the basis of selection for dihydrofolate transferase (dhfr), which confers resistance to methotrexate; xanthine-guanine phosphoribosyl transferase (gpt), which confers resistance to mycophenolic acid; neomycin (neo), which confers resistance to aminoglycoside G-418; and hygromycin B phosphotransferase (hygro) which confers resistance to hygromycin. In a preferred embodiment of the invention, the cell culture is transformed at a low ratio of vector to cell such that there will be only a single vector, or a limited number of vectors, present in any one cell.

### 5.3. USES AND ADMINISTRATION OF TRANS-SPLICING MOLECULES

### 5.3.1. USE OF PTM MOLECULES FOR GENE REGULATION, GENE REPAIR AND TARGETED CELL DEATH

Trans-splicing has various uses beyond that for correcting FVIII genetic defects which forms the focus of the present invention. For example, trans-splicing can be used to introduce a protein with toxic properties into a cell. In addition, PTMs can be engineered to bind to viral mRNA and destroy the function of the viral mRNA, or alternatively, to destroy any cell expressing the viral mRNA. In yet another embodiment of the invention, PTMs can be engineered to place a stop codon in a deleterious mRNA transcript, thereby, decreasing the expression of that transcript. Targeted *trans*-splicing, including double-trans-splicing reactions, 3'exon replacement and/or 5'exon replacement can be used to repair or correct transcripts that are either truncated or contain point mutations. The PTMs may be designed to cleave a targeted transcript upstream or downstream of a specific mutation or upstream of a premature 3' and correct the mutant transcript via a trans-splicing reaction which replaces the portion of the transcript containing the mutation with a functional sequence.

Trans-splicing may also be used to regulate gene expression in plants. For example, trans-splicing may be used to place the expression of any engineered gene under the natural regulation of a chosen target plant gene, thereby regulating the expression of the engineered gene. Trans-splicing may also be used to prevent the expression of engineered genes in non-host plants or to convert an endogenous gene product into a more desirable or toxic (to pest) product.

Trans-splicing may also be used to regulate the expression of the insecticidal gene that produces Bt toxin (Bacillus thuringiensis). For example, the PTM may be designed to trans-splice into an injury response gene (pre-mRNA) that is expressed only after an insect bites the plant. Thus, all cells of the plant would carry the gene for Bt in the PTM, but the cells would only produce Bt when and where an insect injures the plant. The rest of the plant will make little or no Bt. A PTM could trans-splice the Bt gene into any chosen gene with a desired pattern of expression. Further, it should be possible to target a PTM so that no Bt is produced in the edible portion of the plant.

One advantage associated with the use of PTMs is that the PTM acquires the native gene control elements of the target gene, thus, reducing the time and effort that might otherwise be spent attempting to identify and reconstitute appropriate regulatory sequences upstream of an engineered gene. Thus, expression of the PTM regulated gene should occur only in those plant cells containing the target pre-mRNA. By targeting a gene not expressed in the edible portion of the plant or in the pollen, trans-splicing can alleviate opposition to genetically modified plants, as consumers would not be eating the proteins made from modified genes. The edible portion of such crops should test negative for genetically modified proteins.

In addition, PTM can be targeted to a unique sequence of the host gene that is not present in other plants. Therefore, even if the gene (DNA) which encodes the PTM jumps to another species of plant, the PTM gene will not have an appropriate target for trans-splicing. Thus, trans-splicing offers a "fail-safe" mode for prevention of gene "jumping" to other plant species: the PTM gene will be expressed only in the engineered host plant, which contains the appropriate target pre-mRNA. Expression in non-engineered plants would not be possible.

Trans-splicing also provides a more efficient way to convert one gene product into another. For example, trans-splicing ribozymes and chimeric oligos can be incorporated into corn genomes to modify the ratio of saturated to unsaturated oils. Trans-splicing can also be used to convert one gene product into another.

However, the present invention is confined to utilization of trans-splicing to correct FVIII genetic defects. Specifically, targeted trans-splicing, including double-trans-splicing reactions, 3' exon replacement and/or 5' exon replacement can be used to repair or correct FVIII transcripts that are either truncated or contain point mutations. The PTMs of the invention are designed to bind to a targeted FVIII transcript upstream or downstream of a specific mutation or upstream of a premature 3' and correct the mutant transcript via a trans-splicing reaction which replaces the portion of the transcript containing the mutation with a functional sequence.

Various delivery systems are known and can be used to transfer the compositions of the invention into cells, e. g. encapsulation in liposomes, microparticles, microcapsules, recombinant cells capable of expressing the composition, receptor-mediated endocytosis (see, e. g. , Wu and Wu, 1987, J. Biol. Chem. 262: 4429-4432), construction of a nucleic acid as part of a retroviral, adenoviral, adeno-associated viral or other vector, injection of DNA, electroporation, calcium phosphate mediated transfection, etc.

The compositions and methods can be used to treat cancer and other serious viral infections, autoimmune disorders, and other pathological conditions in which alteration or elimination of a specific cell type would be beneficial. Additionally, the compositions and methods can be used to provide a gene encoding a functional biologically active molecule to cells of an individual with an inherited genetic disorder where expression of the missing or mutant gene product produces a normal phenotype.

PTMs of the invention can be used to provide sequences encoding a functional biologically active FVIII molecule to cells of an individual with an inherited genetic disorder where expression of the missing or mutant FVIII gene product produces a normal phenotype, i. e. blood clotting.

In a preferred embodiment, nucleic acids comprising a sequence encoding a PTM are administered to promote PTM function, by way of gene delivery and expression into a host cell. In this embodiment of the invention, the nucleic acid mediates an effect by promoting PTM production. Any of the methods for gene delivery into a host cell available in the art can be used according to the present invention. For general reviews of the methods of gene delivery see Strauss, M. and Barranger, J. A. , 1997, Concepts in Gene Therapy, by Walter de Gruyter & Co., Berlin; Goldspiel et al., 1993, Clinical Pharmacy 12: 488-505; Wu and Wu, 1991, Biotherapy 3: 87-95; Tolstoshev, 1993, Ann. Rev. Pharmacol. Toxicol. 33: 573-596; Mulligan, 1993, Science 260: 926-932; and Morgan and Anderson, 1993, Ann. Rev. Biochem. 62: 191-217; 1993, TIBTECH 11 (5): 155-215. Exemplary methods are described below.

Delivery of the PTM into a host cell may be either direct, in which case the host is directly exposed to the PTM or PTM encoding nucleic acid molecule, or indirect, in which case, host cells are first transformed with the PTM or PTM encoding nucleic acid molecule *in vitro,* then transplanted into the host. These two approaches are known, respectively, as *in vivo* or *ex vivo* gene delivery.

In a specific embodiment, the nucleic acid is directly administered in vivo, where it is expressed to produce the PTM. This can be accomplished by any of numerous methods known in the art, e. g., by constructing it as part of an appropriate nucleic acid expression vector and administering it so that it becomes intracellular, e. g. by infection using a defective or attenuated retroviral or other viral vector (see U. S. Patent No. 4,980, 286), or by direct injection of naked DNA, or by use of microparticle bombardment (e. g., a gene gun; Biolistic, Dupont, Bio-Rad), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see *e.g*., Wu and Wu, 1987, J. Biol. Chem. 262:4429-4432).

In a specific embodiment, a viral vector that contains the PTM can be used. For example, a retroviral vector can be utilized that has been modified to delete retroviral sequences that are not necessary for packaging of the viral genome and integration into host cell DNA (see Miller et al., 1993, Meth. Enzymol. 217:581-599). Alternatively, adenoviral or adeno-associated viral vectors can be used for gene delivery to cells or tissues. (See, Kozarsky and Wilson, 1993, Current Opinion in Genetics and Development 3:499-503 for a review of adenovirus-based gene delivery).

In a preferred embodiment of the invention an adeno-associated viral vector may be used to deliver nucleic acid molecules capable of encoding the PTM. The vector is designed so that, depending on the level of expression desired, the promoter and/or enhancer element of choice may be inserted into the vector.

Another approach to gene delivery into a cell involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. The resulting recombinant cells can be delivered to a host by various methods known in the art. In a preferred embodiment, the cell used for gene delivery is autologous to the host's cell.

In a specific embodiment of the invention, hepatic stem cells, oval cells, or hepatocytes may be removed from a subject having a bleeding disorder and transfected with a nucleic acid molecule capable of encoding a PTM designed to correct a FVIII genetic disorder. Cells may be further selected, using routine methods known to those of skill in the art, for integration of the nucleic acid molecule into the genome thereby providing a stable cell line expressing the PTM of interest. Such cells are then transplanted into the subject thereby providing a source of FVIII protein.

The present invention also provides for pharmaceutical compositions comprising an effective amount of a PTM or a nucleic acid encoding a PTM, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Examples of suitable pharmaceutical carriers are described in "Remington's Pharmaceutical sciences" by E.W. Martin.

In specific embodiments, pharmaceutical compositions are administered: (1) in diseases or disorders involving an absence or decreased (relative to normal or desired) level of an endogenous protein or function, for example, in hosts where the protein is lacking, genetically defective, biologically inactive or underactive, or under expressed; or (2) in diseases or disorders wherein, *in vitro* or *in vivo*, assays indicate the utility of PTMs that inhibit the function of a particular protein. The activity of the protein encoded for by the chimeric mRNA resulting from the PTM mediated *trans*-splicing reaction can be readily detected, *e.g*., by obtaining a host tissue sample (*e.g*., from biopsy tissue) and assaying it *in vitro* for mRNA or protein levels, structure and/or activity of the expressed chimeric mRNA.

In specific embodiments, pharmaceutical compositions are administered in diseases or disorders involving an absence or decreased (relative to normal or desired) level of an endogenous FVIII protein or function, for example, in hosts where the FVIII protein is lacking, genetically defective, biologically inactive or underactive, or under expressed. Such disorders include but are not limited to hemophilia A. The activity of the FVIII protein encoded for by the chimeric or composite mRNA resulting from the PTM mediated *trans*-splicing reaction can be readily detected, *e.g*., by obtaining a host tissue sample (*e.g*., from biopsy tissue) and assaying it *in vitro* for mRNA or protein levels, structure and/or activity of the expressed chimeric mRNA.

Many methods standard in the art can be thus employed, including but not limited to immunoassays to detect and/or visualize the protein encoded for by the chimeric mRNA (*e.g*., Western blot, immunoprecipitation followed by sodium dodecyl sulfate polyacrylamide gel electrophoresis, immunocytochemistry, etc.) and/or hybridization assays to detect formation of chimeric mRNA expression by detecting and/or visualizing the presence of chimeric mRNA (e. g. , Northern assays, dot blots, in situ hybridization, and Reverse-Transcription PCR, etc.), etc.

In a specific embodiment, it may be desirable to administer the pharmaceutical compositions of the invention locally to the area in need of treatment, i. e. , liver tissue. This may be achieved by, for example, and not by way of limitation, local infusion during surgery, topical application, e. g., in conjunction with a wound dressing after surgery, by injection, by means of a catheter, by means of a suppository, or by means of an implant, said implant being of a porous, non-porous, or gelatinous material, including membranes, such as sialastic membranes, or fibers.
Other control release drug delivery systems, such as nanoparticles, matrices such as controlled-release polymers, hydrogels.

The PTM will be administered in amounts which are effective to produce the desired effect in the targeted cell. Effective dosages of the PTMs can be determined through procedures well known to those in the art which address such parameters as biological half-life, bioavailability and toxicity. The amount of the composition of the invention which will be effective will depend on the severity of the clotting disorder being treated, and can be determined by standard clinical techniques. Such techniques include analysis of blood samples to determine clotting time. In addition, in vitro assays may optionally be employed to help identify optimal dosage ranges.

The present invention also provides a pharmaceutical pack or kit comprising one or more containers filled with one or more of the ingredients of the pharmaceutical compositions of the invention optionally associated with such container (s) can be a notice in the form prescribed by a governmental agency regulating the manufacture, use or sale of pharmaceuticals or biological products, which notice reflects approval by the agency of manufacture, use or sale for human administration.

### 6. EXAMPLE: PRODUCTION OF EFFICIENTLY TRANS-SPLICING MOLECULES

The following section describes PTM molecules that have been designed to increase the efficiency of trans-splicing. The PTMs contain one or more of the following features (i) a binding domain of a 5'exon replacement PTM that is targeted to intron sequences in close proximity to the 3' splice signals of the intron; (ii) mini-intron sequences inserted into the coding region of the PTM; (iii) ISAR (intronic splicing activator and repressor) consensus binding sites inserted downstream but in close proximity to the PTM donor site; and/or a ribozyme sequence.

### 6.1. MATERIALS AND METHODS

### 6.1. 1. PLASMID CONSTRUCTION

The LacZ coding sequences for each PTM obtained either by PCR or by subcloning a fragment from an existing LacZ mini-gene target. The trans-splicing domain (TSD) including the binding domain, spacer sequence, and 5' splice site was generated from a PCR product using an existing plasmid template and by annealing oligonucleotides. The different fragments (the TSD and coding sequences) were then cloned into pcDNA3.1 (-) using appropriate restriction sites. Oligodeoxynucleotide primers were procured from Sigma Genosys (The Woodlands, TX). All PCR products were generated with either REDTaq (Sigma, St. Louis, MO), or cloned Pfu (Stratagene, La Jolla, CA) DNA Polymerase. PCR primers for amplification contained restriction sites for directed cloning. PCR products were digested with the appropriate restriction enzymes and cloned into the mammalian expression plasmid pc3. 1DNA (-) (Invitrogen, Carlsbad, CA).

### 6.1. 2. CELL CULTURE AND TRANSFECTIONS

Constructs were cotransfected into human embryonic kidney (HEK) 293T (1.5 x 10⁶ cells per 60 mm poly-d-lysine coated dish) using LipofectaminePlus (Life Technologies, Gaithersburg, MD) and the cells were harvested 48 h after the start of transfection. HEK 293T cells were grown in Dulbecco's Modified Eagle's Medium (Life Technologies) supplemented with 10% v/v fetal bovine serum (Hyclone, Inc. , Logan, UT). All cells were kept in a humidified incubator at 37 C and 5% CO2.

### 6.1. 3. GALACTOSIDASE IN SOLUTION ASSAY AND IN-SITU STAINING

Total cellular protein from cells transfected with expression plasmids was isolated by a freeze thaw method and assayed for p-galactosidase activity using a (3-gal assay kit (Invitrogen, Carlsbad, CA). Protein concentration was measured by the dye-binding assay using Bio-Rad protein assay reagents (BIO-RAD, Hercules, CA). Cells were monitored for the expression of functional -galactosidase using a p-gal staining kit (Invitrogen, Carlsbad, CA).

### 6.2. RESULTS

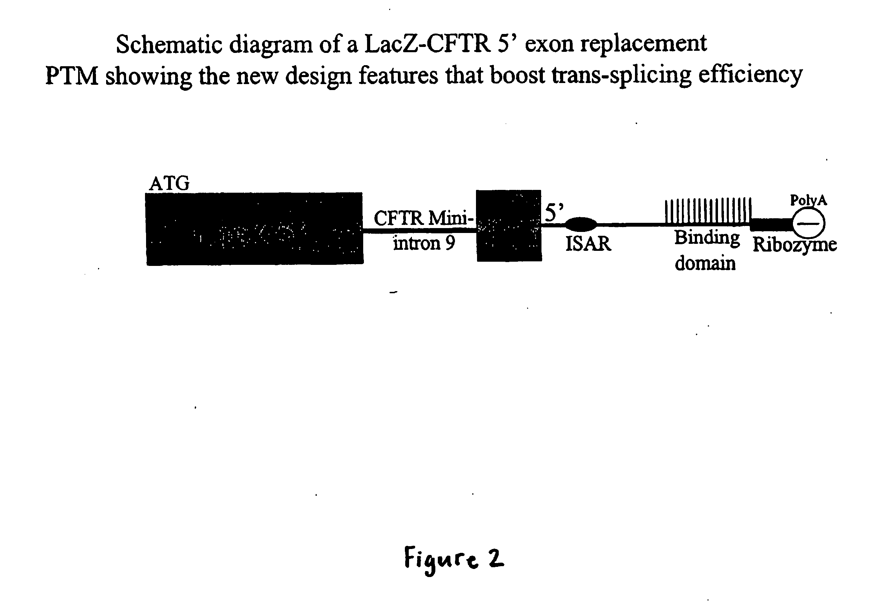

A number of novel PTM features were found to enhance the trans- splicing of PTMs to target pre-mRNA molecules. A schematic diagram of a 5'LacZ construct showing the new design features is shown in Figure 2. Details of the mutant LacZ target used in assessing repair efficiency for each new construct is shown in Figure 3.

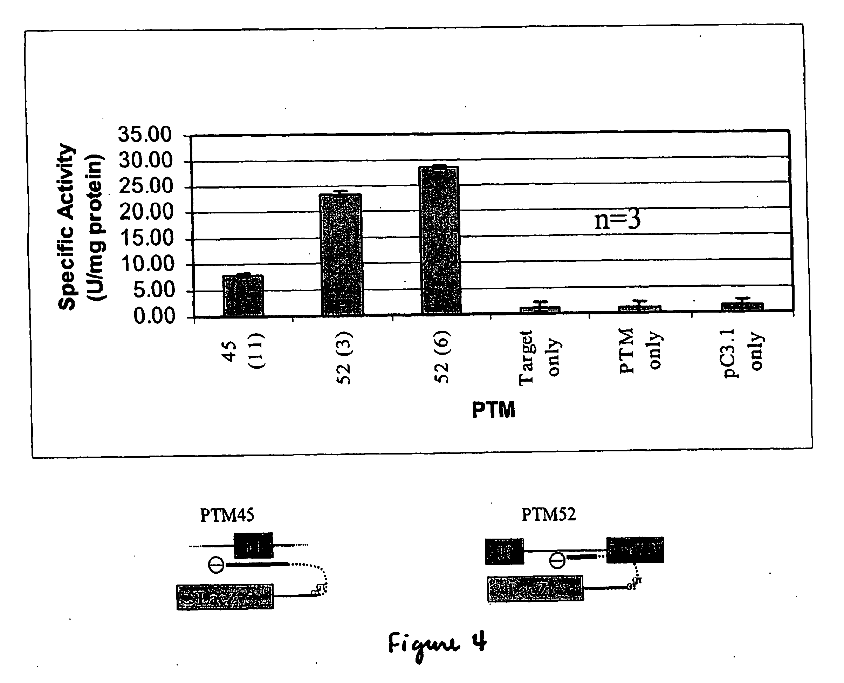

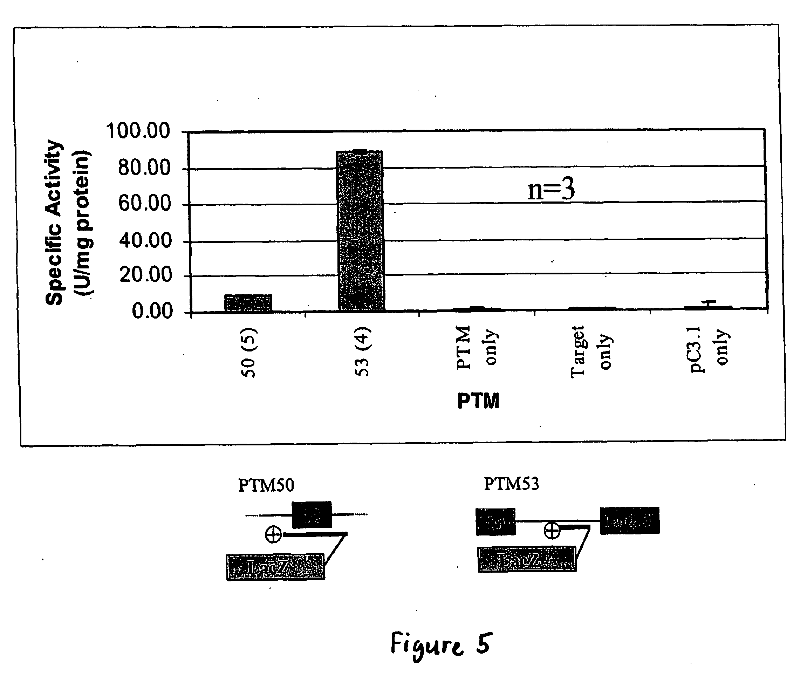

A PTM binding domain targeted to bind to intron sequences in close proximity to the 3' splice sequences of the intron was found to increase the efficiency of trans-splicing. This particular design feature is unique because in previous PTM constructs the constructs were designed to occlude the 5' splice site of the same intron. Targeting of the PTM to the 3' end of an intron is intended to bring the PTM donor site in close proximity to the target acceptor site (see Figure 4 and 5).

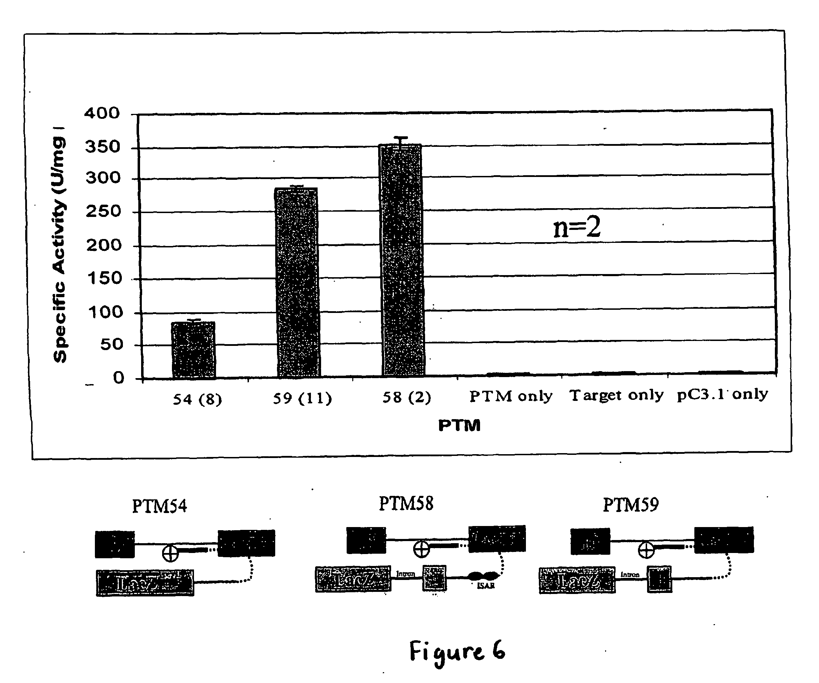

Inclusion of a mini-intron into the coding sequence of a PTM is designed to increase exon definition and enhance recognition of the PTM donor site. A mini- intron, approximately 400 bp in length was generated by fusing the functional end of CFTR intron 9. The mini intron was inserted into a PTM and tested for splicing efficiency. As demonstrated in Figure 6, splicing efficiency was enhanced using PTMs having a mini-intron insert.

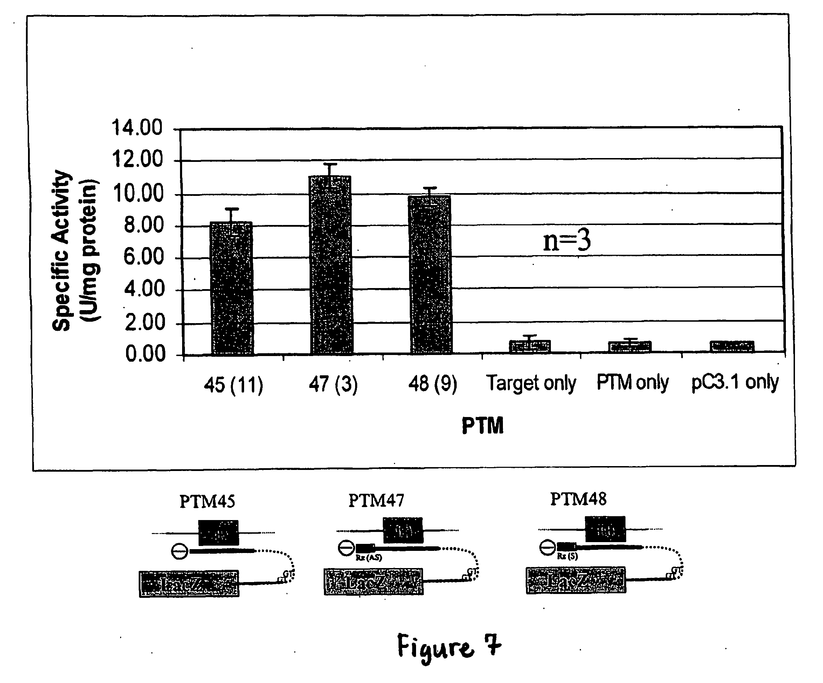

A protein, referred to as the ISAR protein, associates selectively with pre-mRNAs that contain 5' splice sites followed by U-rich sequences ("ISAR sequences"). ISAR binding to the U-rich regions is believed to facilitate 5' splice site recognition by U1 snRNP. As demonstrated in Figure 6, PTMs containing ISAR sequences were capable of mediating trans-splicing reactions with a higher efficiency than those PTMs not containing an ISAR sequence. A cis-acting ribozyme was inserted at the end of the PTM trans-splicing domain and tested for trans-splicing. The ribozyme sequences were included in the PTM structure to reduce PTM translation in the absence of trans-splicing. As indicated in Figure 7, the inclusion of ribozyme sequences in the structure of PTMs increased the efficiency of trans- splicing.

### 7. EXAMPLE: CORRECTION OF THE FACTOR VIII GENE USING 3'EXON REPLACEMENT

Hemophilia is a bleeding disorder caused by a deficiency in one of the blood clotting factors. Hemophilia A, which accounts for about 80 percent of all cases is caused by a deficiency in clotting factor VIII. The following section describes the successful repair of the clotting factor VIII gene using spliceosome mediated trans-splicing and demonstrates the feasibility of repairing the factor VIII using gene therapy.

The coding region for mouse factor VIII PTM (exons 16-24) was PCR amplified from a cDNA plasmid template using primers that included unique restriction sites for directed cloning. All PCR products were generated with cloned Pfu DNA Polymerase (Stratagene, La Jolla, CA). The coding sequence was cloned into pc3. 1 DNA (-) using EcoRV and PmeI restriction sites. The binding domain (BD) was created by PCR using genomic DNA as a template. Primers included unique restriction sites for directed cloning. The PCR product was cloned into an existing PTM plasmid (PTM-CF24, pc3. 1DNA) using NheI and SacII restriction sites. This plasmid already contained the remaining elements of the TSD including a spacer sequence, polypyrimidine tract (PPT), branchpoint (BP) and 3' acceptor site. The whole of the TSD was then subcloned into the vector (described above) containing the factor VIII PTM coding sequences. Finally, bovine growth hormone 3'untranslated sequences from a separate plasmid clone were subcloned into the above PTM using PmeI and BamHI restriction sites.

The whole construct was sequenced and then analyzed by RT-PCR for possible cryptic splicing, and then subcloned into the AAV plasmid pDLZ20-M2 using XhoI and BamHI restriction sites (Chao et al., 2000, Gene Therapy 95: 1594- 1599; Flotte and Carter, 1998, Methods Enzymol., 292: 717-32). For some viral (and non-viral) delivery systems, the size of the therapeutic is essential. Viral vectors such as adeno-associated virus are preferred because they are a (i) non-pathogenic virus with a broad host range (ii) they induce a low inflammatory response when compared to adenovirus vectors and (iii) it has the ability to infect both dividing and non- dividing cells. However, the packaging capacity of the rAAV is limited to approximately 110% of the size of the wild type genome, or-4. 9 kB, thus, leaving little room for large regulatory elements such as promoters and enhancers. The B- domain deleted human Factor VIII is close to the packaging size of AAV, thus, trans- splicing offers the possibility of delivering a smaller transgene while permitting the addition of regulatory elements.

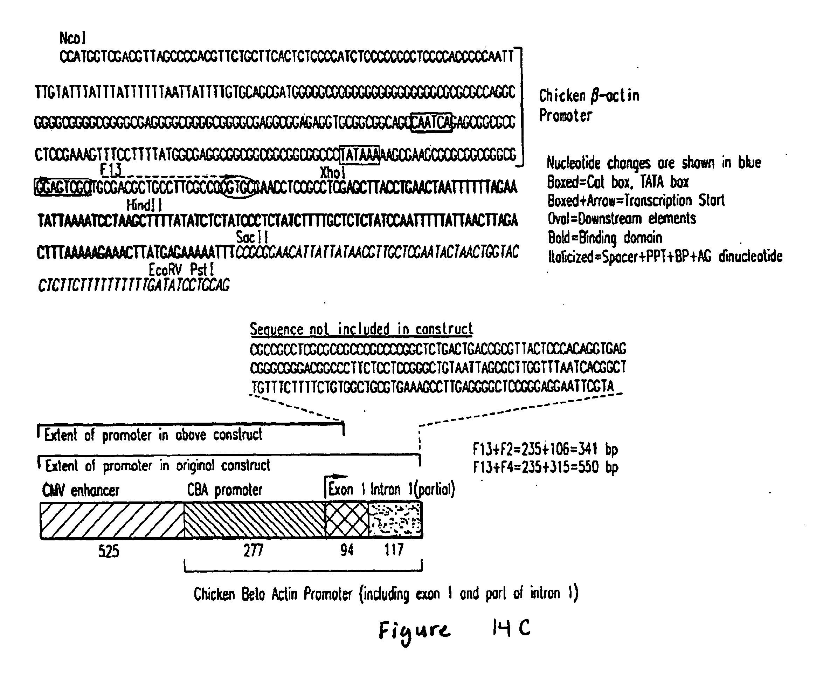

To eliminate cryptic donor sites in the pre-mRNA upstream of the XhoI PTM cloning site approximately 170 bp of sequence was eliminated from the original AAV construct that includes part of exon 1 and all of the intron 1 sequence (see Fig. 14C).

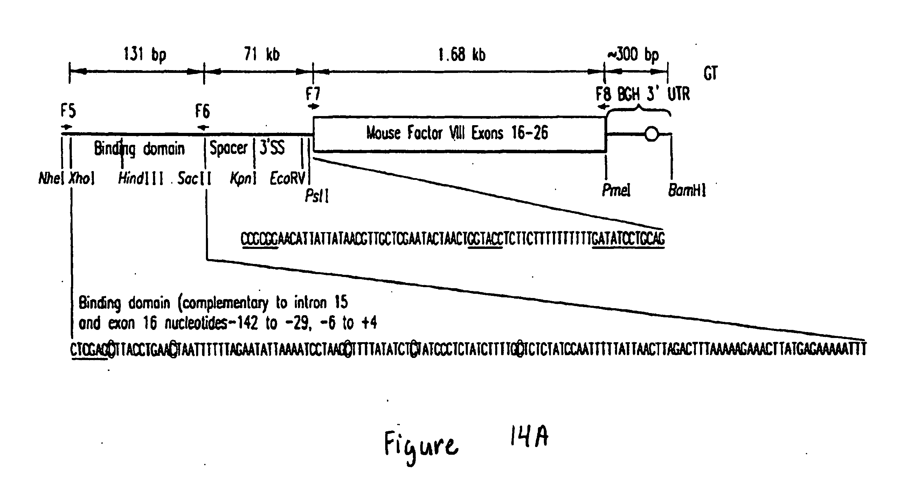

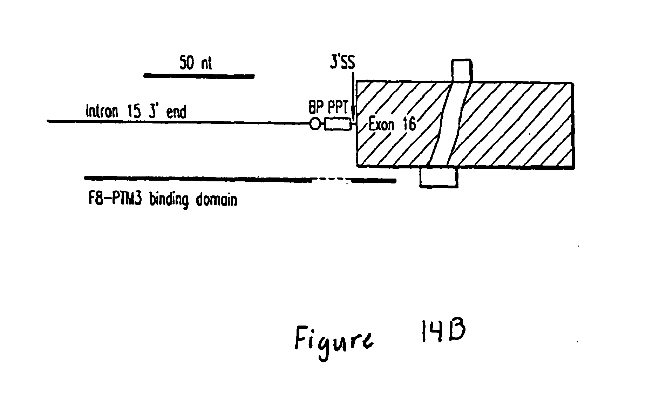

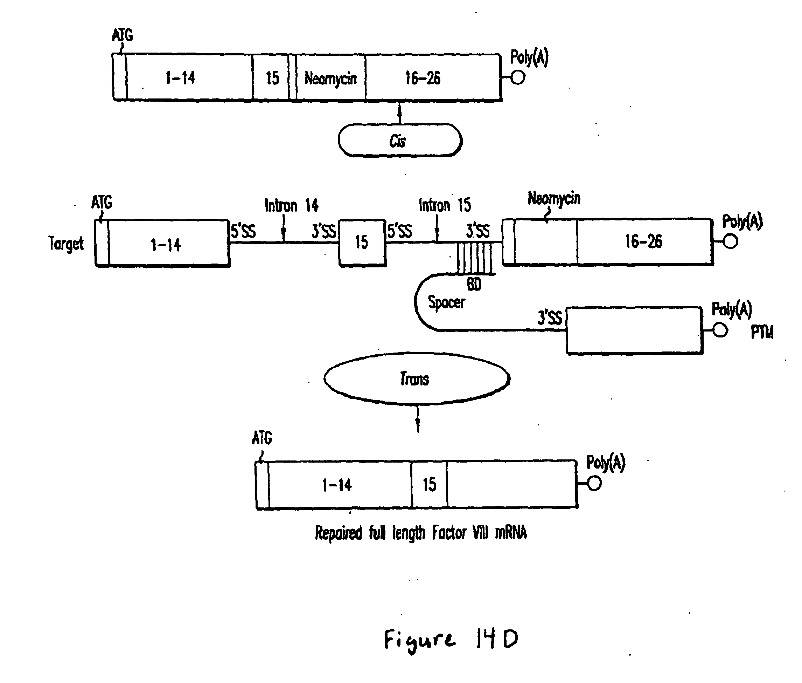

The repair model in Fig. 14D shows a simplified model of the mouse factor VIII pre-mRNA target (endogenous gene) consisting of exons 1-14, intron 14, exon 15, intron 16, and exon 16-26 containing a neomycin gene insertion. The PTM shown in the figure consists of exon 16-26 coding sequences and a trans-splicing domain with its own splicing elements (donor site, branchpoint and pyrimidine tract) and a binding domain. Details of the binding domain are shown in Fig. 14A and 14B. The binding domain is complementary to the splice site of intron 15 and part of exon 16 (5' end).

The key advantages of using 3'exon replacement for gene repair are (i) the construct requires less sequence and space than a full length gene construct, thereby leaving more space for regulatory elements, (ii) trans-splicing repair should only occur in those cells that express the target gene, therefore eliminating any potential problems associated with ectopic expression of repaired RNA, and (iii) trans-splicing generates a full-length mRNA that includes the B-domain.

For plasmid injections each FVIII deficient mouse was sedated and placed under a dissecting microscope and a 1 cm vertical midline abdomen incision was made. Approximately 100 micrograms of PTM plasmid DNA in phosphate buffered saline was injected to liver portal vein. Blood was collected from the retro- orbital plexus at intervals of 1,2, 3 and 20 days after injection and assayed for Factor VIII activity using the Coatest assay.

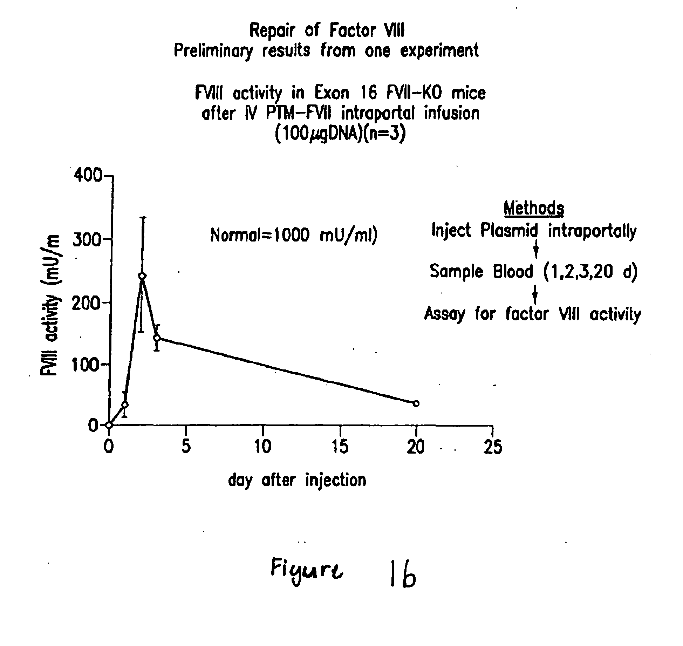

Factor VIII activity in blood samples collected from mice were assayed using a standard test called the Coatest assay. The assay was performed according to manufacturer's instructions (Chromgenix AB, Milan, Italy). Data indicating repair of factor VIII in factor VIII knock out mice is demonstrated in Figure 16.

Hemophilia A defects in humans are broadly split into several categories that include gross DNA rearrangements, single DNA base substitutions, deletions and insertions. It has been determined that a rearrangement of DNA involving an inversion and translocation of exons 1-22 (together with introns) away from exons 23-26 is responsible for-40% of all cases of severe hemophilia A. The canine hemophilia A model also has a very similar gross rearrangement. This mutation is an important consideration in the deisgn of human and canine factor VIII PTM.

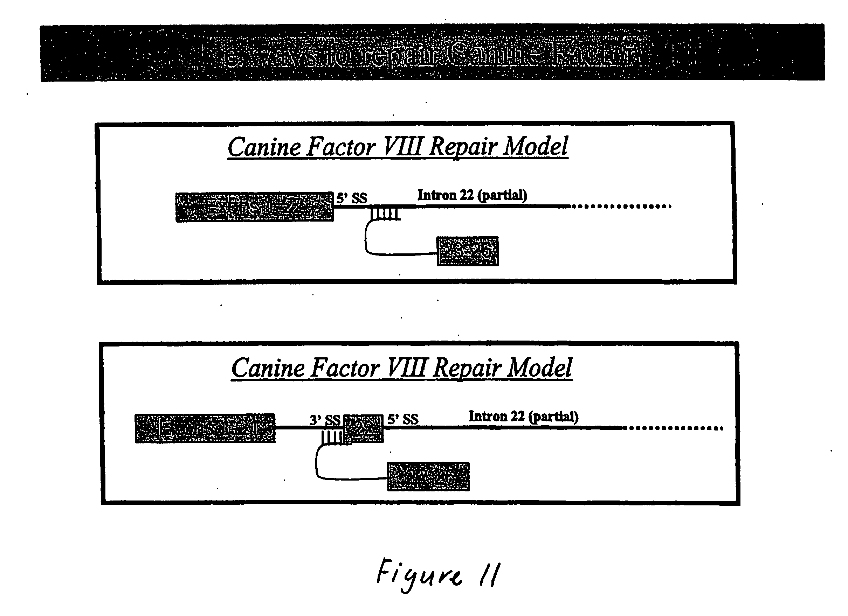

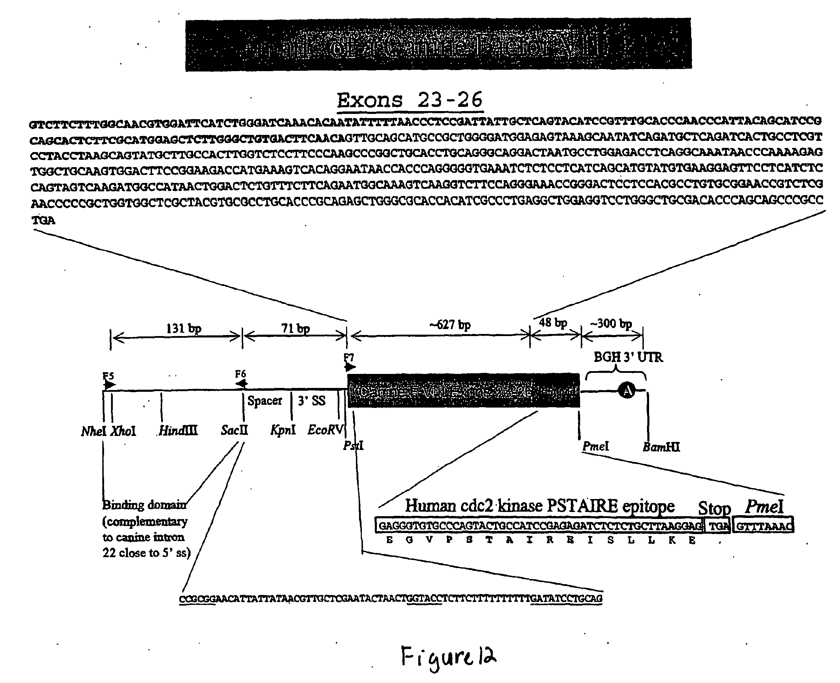

Methods for building the human Factor VIII PTM will be very similar to that described above for the mouse PTM except that different coding regions (such as exons 15-26) will be amplified from a human cDNA, the binding domain will be amplified from human genomic sequence templates (whole genomic DNA or a genomic clone), and a C-terminal tag may be engineered in the PTM to facilitate detection of repaired Factor VIII protein. The remaining elements of the trans- splicing domain including a spacer sequence, polypyrimidine tract (PPT), branchpoint (BP) and 3' acceptor site will be obtained from an existing PTM. Where necessary changes will be made to the binding domain sequence to eliminate cryptic splicing within the PTM. The final PTM will be subcloned into an AAV plasmid vector, such as pDLZ20-M2. Virus can be prepared from this plasmid. A canine Factor VIII PTM can be made using these design methods by incorporating canine cDNA and genomic plasmids (See, Figures 11 and 12).

## Claims

1. A nucleic acid molecule comprising
a) one or more target-binding domains that target binding of the nucleic acid to a human factor VIII target pre-mRNA expressed within a cell;
b) (1) a 3' splice region comprising a 3' splice acceptor site, a branch point and a polypyrimidine tract and/or (2) a 5' splice donor site;
c) a spacer region that separates the 3' splice region or 5' splice donor site from the target-binding domain; and
d) a nucleotide sequence of (1) human factor VIII exons 15 to 26, 16 to 26, 17 to 26, 18 to 26, 19 to 26, 20 to 26, 21 to 26 or 22 to 26, to be trans-spliced to the target pre-mRNA (for mediating 3' exon replacement in the pre-mRNA), or (2) human factor VIII exons 1 alone, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14 or 1 to 15 (for mediating 5' exon replacement in the pre-mRNA), said nucleic acid molecule being recognised by nuclear splicing components within the cell.

2. A nucleic acid molecule according to Claim 1, which further comprises a safety sequence having one or more complementary sequences that bind to one or both sides of the 3' splice region and/or one or both sides of the 5' splice site.

3. An expression vector which expresses a nucleic acid molecule claimed in Claim 1 or 2.

4. A vector according to Claim 3 in the form of a viral vector.

5. A vector according to Claim 4 which is an adeno-associated virus (AAV), adenovirus or retrovirus vector.

6. A composition comprising a physiologically acceptable carrier and a nucleic acid molecule claimed in Claim 1 or 2.

7. A nucleic acid molecule according to Claim 1 or 2 or a vector according to Claim 3, 4, or 5, for use in correcting in a human subject a genetic defect in factor VIII by introducing said nucleic acid molecule or vector into target cells of the human subject expressing a human Factor VIII target pre-mRNA.

8. A nucleic acid molecule according to Claim 7 for use in correcting in a human subject a genetic defect in factor VIII wherein host cells are first transformed with said nucleic acid molecule or vector *in vitro* and then transplanted into the subject.

9. A nucleic acid molecule or vector according to Claim 7 for use in correcting in a human subject a genetic defect in factor VIII wherein the target cells of the subject are directly exposed to said nucleic acid molecule or vector *in vivo.*

## Patentansprüche

1. Nukleinsäuremolekül, umfassend
a) eine oder mehrere zielbindende Domänen, die auf die Bindung der Nukleinsäure an eine Humanfaktor-VIII-Ziel pre-mRNA abzielt, die innerhalb einer Zelle exprimiert wird;
b) (1) eine 3'-Spleißregion, umfassend eine 3'-Spleißakzeptorstelle, einen Verzweigungspunkt und einen Polypyrimidintrakt und/oder (2) eine 5'-Spleißdonorstelle;
c) eine Abstandregion, welche die 3'-Spleißregion oder die 5'-Spleißdonorstelle von der zielbindenden Domäne trennt; und
d) eine Nukleotidsequenz von (1) Humanfaktor-VII-Exonen 15 bis 26, 16 bis 26, 17 bis 26, 18 bis 26, 19 bis 26, 20 bis 26, 21 bis 26 oder 22 bis 26, die an die Ziel-pre-mRNA transgespleißt werden sollen (zur Vermittlung eines 3'-Exon-Ersatzes in der pre-mRNA) oder (2) des Humanfaktors-VIII-Exons 1 allein, 1 bis 2, 1 bis 3, 1 bis 4, 1 bis 5, 1 bis 6, 1 bis 7, 1 bis 8, 1 bis 9, 1 bis 10, 1 bis 11, 1 bis 12, 1 bis 13, 1 bis 14 oder 1 bis 15 (zur Vermittlung des 5'-Exon-Ersatzes in der pre-mRNA), wobei das genannte Nukleinsäuremolekül von nuklearen Spleißkomponenten innerhalb der Zelle erkannt wird.

2. Nukleinsäuremolekül nach Anspruch 1, ferner umfassend eine Sicherheitssequenz mit einer oder mehreren komplementären Sequenzen, die sich an eine oder beide Seiten der 3'-Spleißregion und/oder an eine oder beide Seiten der 5'-Spleißstelle binden.

3. Expressionsvektor, der ein in Anspruch 1 oder 2 beanspruchtes Nukleinsäuremolekül exprimiert.

4. Vektor nach Anspruch 3 in Form eines Virusvektors.

5. Vektor nach Anspruch 4, der ein Adeno-assoziierter Virus (AAV), Adenovirus- oder Retrovirusvektor ist.

6. Zusammensetzung umfassend einen physiologisch akzeptablen Träger und ein in Anspruch 1 oder 2 beanspruchtes Nukleinsäuremolekül.

7. Nukleinsäuremolekül nach Anspruch 1 oder 2 oder ein Vektor nach Anspruch 3, 4 oder 5 zur Verwendung bei der Korrektur eines genetischen Defekts des Faktors VIII in einer menschlichen Testperson durch Einführen des genannten Nukleinsäuremoleküls oder Vektors in die Zielzellen der menschlichen Testperson, die eine Humanfaktor-VIII-Ziel-pre-mRNA exprimieren.

8. Nukleinsäuremolekül nach Anspruch 7 zur Verwendung bei der Korrektur eines genetischen Defekts des Faktors VIII in einer menschlichen Testperson, wobei Wirtzellen zuerst mit dem genannten Nukleinsäuremolekül oder Vektor *in vitro* transformiert und dann in die Testperson transplantiert werden.

9. Nukleinsäuremolekül oder Vektor nach Anspruch 7 zur Verwendung bei der Korrektur eines genetischen Defekts des Faktors VIII in einer menschlichen Testperson, wobei die Zielzellen der Testperson dem genannten Nukleinsäuremolekül oder dem Vektor *in vivo* direkt ausgesetzt werden.

## Revendications

1. Molécule d'acide nucléique comprenant
a) un ou plusieurs domaines de liaison de cible qui ciblent la liaison de l'acide nucléique à un pré-ARNm cible de facteur VIII humain exprimé dans une cellule ;
b) (1) une région d'épissage en 3' comprenant un site accepteur d'épissage en 3', un point de branchement et un tractus polypyrimidine et/ou (2) un site donneur d'épissage en 5';
c) une région d'espaceur qui sépare la région d'épissage en 3' ou le site donneur d'épissage en 5' du domaine de liaison de cible ; et
d) une séquence nucléotidique de (1) exons de facteur VIII humain 15 à 26, 16 à 26, 17 à 26, 18 à 26, 19 à 26, 20 à 26, 21 à 26 ou 22 à 26, destinée à être épissée en trans au pré-ARNm cible (pour la médiation du remplacement de l'exon en 3' dans le pré-ARNm), ou (2) les exons de facteur VIII humain 1 uniquement, 1 à 2, 1 à 3, 1 à 4, 1 à 5, 1 à 6. 1 à 7, 1 à 8, 1 à 9, 1 à 10, 1 à 11, 1 à 12, 1 à 13, 1 à 14 ou 1 à 15 (pour la médiation du remplacement de l'exon en 5' dans le pré-ARNm), ladite molécule d'acide nucléique étant reconnue par le composant d'épissage nucléaire dans la cellule.

2. Molécule d'acide nucléique selon la revendication 1, qui comprend en outre une séquence de sécurité ayant une ou plusieurs séquences complémentaires qui se lient à un ou aux deux côtés de la région d'épissage en 3' et/ou à un ou aux deux côtés de la région d'épissage en 5'.

3. Vecteur d'expression qui exprime une molécule d'acide nucléique selon la revendication 1 ou 2.

4. Vecteur selon la revendication 3 sous la forme d'un vecteur viral.

5. Vecteur selon la revendication 4 qui est un vecteur à virus adéno-associé (AAV), adénovirus ou rétrovirus.

6. Composition comprenant un véhicule physiologiquement acceptable et une molécule d'acide nucléique selon la revendication 1 ou 2.

7. Molécule d'acide nucléique selon la revendication 1 ou 2 ou vecteur selon la revendication 3, 4, ou 5, pour utilisation dans la correction chez un sujet humain d'un défaut génétique dans le facteur VIII par introduction de ladite molécule d'acide nucléique ou dudit vecteur dans des cellules cibles du sujet humain exprimant un pré-ARNm cible de facteur VIII humain.

8. Molécule d'acide nucléique selon la revendication 7 pour utilisation dans la correction chez un sujet humain d'un défaut génétique dans le facteur VIII où des cellules hôtes sont dans un premier temps transformées avec ladite molécule d'acide nucléique ou ledit vecteur *in vitro* et ensuite transplantées dans le sujet.

9. Molécule d'acide nucléique ou vecteur selon la revendication 7 pour utilisation dans la correction chez un sujet humain d'un défaut génétique dans le facteur VIII où les cellules cibles du sujet sont directement exposées à ladite molécule d'acide nucléique ou au vecteur *in vivo.*
